# EUROPEAN PATENT SPECIFICATION

(11) **EP 1 243 595 B1**
(45) Date of publication and mention of the grant of the patent: **06.09.2006**
(21) Application number: 00985913.3
(22) Date of filing: 26.12.2000
(51) Int. Cl.: C07K 14/47, C12N 15/12, A61K 38/17, A61P 9/10, C12N 15/62

(54) **BH4-FUSED POLYPEPTIDES**
MIT BH4 FUSIONIERTE POLYPEPTIDE
POLYPEPTIDES BH4-FUSIONNES

(30) Priority: 27.12.1999 JP 37144999
(43) Date of publication of application: 25.09.2002
(73) Proprietor: SHIONOGI & CO., LTD., Osaka-shi, Osaka 541-0045 (JP)
(72) Inventor: SHIMIZU, Shigeomi, Osaka-shi, Osaka 553-0003 (JP); TSUJIMOTO, Yoshihide, Toyonaka-shi, Osaka 560-0002 (JP)
(74) Representative: Vossius & Partner
(86) International application number: PCT/JP2000/009274
(87) International publication number: WO 2001/048014

(56) References cited:
- US-A- 5 834 309
- XIU-HUAI LIU, JOANN C. CASTELLI, AND RICHARD J. YOULE: "Receptor-mediated uptake of an extracellular Bcl-xL fusion protein inhibits apoptosis" PROCEEDINGS OF THE NATIONAL ACADEMY OF SCIENCES USA, US, vol. 96, 17 August 1999 (1999-08-17), pages 9563-9567, XP002282327
- DAVID C.S. HUANG, JERRY M. ADAMS AND SUZANNE CORY: "The conserved N-terminal BH4 domain of Bcl-2 homologues is essential for inhibition of apoptosis and interaction with CED-4" THE EMBO JOURNAL, vol. 17, 1998, pages 1029-1039, XP002282328
- HIROTANI M ET AL: "NH2-TERMINAL BH4 DOMAIN OF BCL-2 IS FUNCTIONAL FOR HETERODIMERIZATION WITH BAX AND INHIBITION OF APOPTOSIS" JOURNAL OF BIOLOGICAL CHEMISTRY, AMERICAN SOCIETY OF BIOLOGICAL CHEMISTS, BALTIMORE, MD, US, vol. 274, no. 29, 16 July 1999 (1999-07-16), pages 20415-20420, XP002936736 ISSN: 0021-9258
- SCHWARZE S R ET AL: "In vivo protein transduction: delivery of a biologically active protein into the mouse" SCIENCE, AAAS. LANCASTER, PA, US, vol. 285, 3 September 1999 (1999-09-03), pages 1569-1572, XP002204883 ISSN: 0036-8075
- JOHNS D G: "GLUCOCORTICOID REGULATION OF TETRAHYDROBIOPTERIN (BH4) SYNTHESIS AND ENDOTHELIUM DEPENDENT VASODILATION" FASEB JOURNAL, FED. OF AMERICAN SOC. FOR EXPERIMENTAL BIOLOGY, BETHESDA, MD, US, vol. 13, no. 4, PART 1, 1999, page A99 XP000891658 ISSN: 0892-6638
- RIE SUGIOKA, SHIGEOMI SHIMIZU, TOSHIHIRO FUNATSU, HIROSHI TAMAGAWA, YOSHIKI SAWA, TORU KAWAKAMI, YOSHIHIDE TSUJIMOTO: "BH4-domain peptide from Bcl-xL exerts anti-apoptotic activity in vivo" ONCOGENE, vol. 22, no. 52, 20 November 2003 (2003-11-20), pages 8432-8440, XP002282330
- S. SHIMIZU ET AL.: 'Bcl-2 family proteins regulate the release of apoptogenic cytochrome C by the mitochondrial channel VDAC' NATURE vol. 399, June 1999, pages 483 - 487, XP002941604
- Y. TSUJIMOTO ET AL.: 'Involvement of the bcl-2 gene in human follicular lymphoma' SCIENCE vol. 228, 1985, pages 1440 - 1443, XP002941605

## Description

### TECHNICAL FIELD

The present invention relates to a BH4 fusion polypeptide capable of inhibiting apoptosis and its use, an apoptosis inhibitor and uses for treating ischemic diseases. More particularly, the present invention relates to a BH4 fusion polypeptide possessing an incorporation action into a cell and capable of inhibiting apoptosis, and its use, an apoptosis inhibitor comprising the fusion polypeptide, and uses for treating ischemic diseases. The BH4 fusion polypeptide is useful as therapeutic agents for treating AIDS, neurodegenerative diseases, osteomyelodysplasia, ischemic diseases, infectious multiple organ failure, fulminant hepatitis, ischemic reperfusion disorders, diabetes and the like.

### BACKGROUND ART

Cell death plays an important role *in vivo* in the dynamics of cells such as organ and tissue cells. Many of the cell deaths progress by apoptosis, and the mechanism of the apoptosis is usually strictly controlled. The above-mentioned apoptosis may also be caused by pathological factors.

Recently, a large number of molecules associated with apoptosis have been identified, and apoptotic signaling mechanisms have been investigated. For example, it has been shown that Bcl-2 and Bcl-x_{L} proteins suppressively regulate apoptotic signaling upstream of caspase, a protease which is essential to induction of apoptosis, and that caspase family acts as a mediator for the signal.

Bcl-2 protein inhibits apoptosis by inhibiting loss of mitochondrial membrane potential (ΔΨ), release of cytochrome *c* from mitochondria, and release of apoptosis-inducing factor (hereinafter referred to as "AIF"). The above-mentioned Bcl-2 protein is the product of bcl-2 gene, which was found as one of oncogenes by Tsujimoto et al. [Tsujimoto, Y. et al., *Science,* **228**: 1440-1443 (1985)].

Recently, there have been suggested that 1) Bcl-2 family protein directly targets voltage-dependent anion channel (VDAC) to regulate apoptotic release of cytochrome *c*; 2) pro-apoptotic factors such as Bax and Bak stimulate VDAC activity to allow for passage of cytochrome *c*, whereas anti-apoptotic factors inhibit the VDAC; and 3) VDAC is required for apoptotic ΔΨ loss to occur. The above-mentioned VDAC, which is abundantly present in mitochondrial outer membrane, has a function of regulating mitochondrial membrane permeability and form a permeability transition pore together with adenine nucleotide translocator (ANT) and other molecule.

As described above, there have been suggested various involvements of Bcl-2 family protein in inhibiting release of cytochrome *c*, ΔΨ loss or the like. However, there are much unclarified matters in the detailed action mechanism at present.

In addition, when an intact Bcl-2 protein is used, there is a defect that the protein becomes large and tends to be degraded, and the production efficiency is lowered in some cases.

### DISCLOSURE OF INVENTION

An object of the present invention is to provide a BH4 fusion polypeptide capable of inhibiting apoptosis and its use as an apoptosis inhibitor and uses for treating ischemic diseases

The present invention relates to:
[1] a Bcl-2 homology 4 domain (BH4) fusion polypeptide consisting of:
   an amino acid sequence of a polypeptide or a derivative sequence thereof capable of exhibiting uptake action into a cell; and
   an amino acid sequence selected from the group consisting of:
      (A) an amino acid sequence of SEQ ID NOs: 1, 2, 3, 4, 9, 14, 15, 16, 17, 18, 19, 20, 21 or Ser 11 to Ser 30 of SEQ ID NO:29; and
      (B) an amino acid sequence having substitution, deletion or insertion of one amino acid residue in the amino acid sequence of SEQ ID NOs:1, 2, 3, 4, 9, 14, 15, 16, 17, 18, 19, 20, 21 or Ser 11 to Ser 30 of SEQ ID NO:29,
   wherein the BH4 fusion polypeptide is capable of inhibiting apoptosis;
[2] a DNA encoding the BH4 fusion polypeptide of item [1] above;
[3] an apoptosis-inhibitor comprising the BH4 fusion polypeptide of item [1] above
[4] a use of the apoptosis-inhibitor of item [3] for the preparation of a pharmaceutical composition for treating an ischemic disease, characterized by administering the ischemic disease, wherein apoptosis is inhibited, thereby the ischemic disease is treated; and
[5] use of the BH4 polypeptide of item [1] above, for manufacturing a prophylactic or therapeutic agent for an ischemic disease.

### BRIEF DESCRIPTION OF THE DRAWINGS

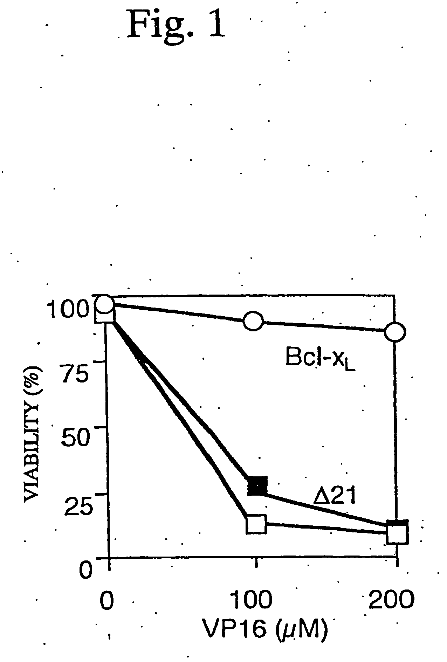

Figure 1 is a diagram showing the results for evaluating the effect of transient expression of BCl-x_{L} and Δ21 on VP16-induced apoptosis. HeLa cells were transfected with a Bcl-x_{L}-expressing construct (O), a Δ21-expressing construct (■) or a vector alone (□) as well as with 0.1 µg of GFP-expressing construct (for detecting DNA-transfected cells) for 24 hours. The transfected cells were treated with VP16 at indicated concentrations for 24 hours. Treated cells were then stained with Hoechst 33342. Apoptotic cell death was determined as a ratio of GFP-positive cells with nuclear fragmentation among all GFP-positive cells. Data shown are from one of three independent experiments.

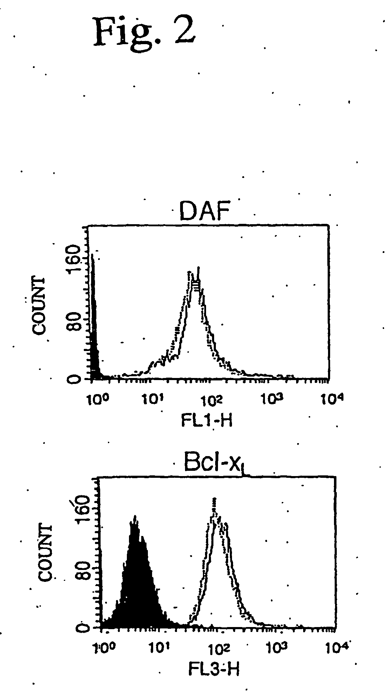

Figure 2 is a diagram showing the results for evaluating the sorting of Bcl-x_{L-}expressing HeLa cells and Δ21-expressing HeLa cells sorted by flow cytometry. HeLa cells were transfected with the product for expression of Bcl-x_{L}, Δ21 or vector alone together with a DAF-expressing plasmid for 24 hours. Next, the cells were stained with anti-human DAF antibody, and DAF-positive cells were sorted by flow cytometry. Expression of DAF (the upper panel) or Bcl-x_{L} and Δ21 proteins (the lower panel) by the sorted cells were assayed by flow cytometry. Dotted lines show the results for Bcl-x_{L}-expressing cell, and solid lines show the results for Δ21-expressing cells: Solid image region represents the results for untransfected cells. Data shown are the representative results of three independent experiments.

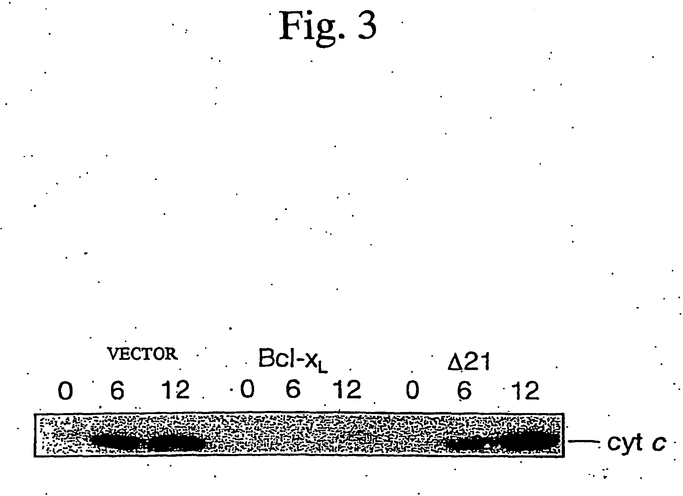

Figure 3 is a diagram showing the results for evaluating the effects of Bcl-x_{L} or Δ21 on apoptotic release of cytochrome c. DAF-positive cells sorted as in Figure 2 were treated with 200 µM VP16 for indicated periods of time, subjected to biochemical cell fractionation and then to Western blotting, to determine release of cytochrome c. Data shown are from one of three independent experiments.

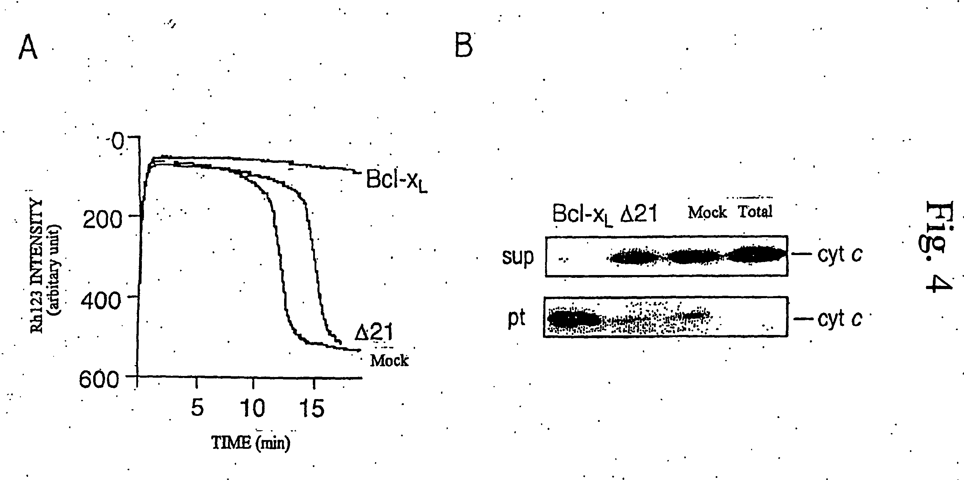

Figure 4 is a diagram showing the results for evaluating the role of BH4 domain of Bcl-x_{L} in the inhibition of Ca²⁺-induced ΔΨ loss and release of cytochrome c from isolated mitochondria. Panel (A) shows the results obtained by treating rat liver mitochondria (1 mg/ml) with rBcl-x_{L}, rΔ21 or mock protein (20 µg/ml) together with 40 µM Ca²⁺, and examining ΔΨ using Rh123. Panel (B) shows the results for evaluating cytochrome c levels in supernatant (sup) and mitochondria (pt) fractions at 15 minutes after incubation. Data shown are from one of three independent experiments. "Total " indicates the total amount of cytochrome c release by 1% Triton-X100.

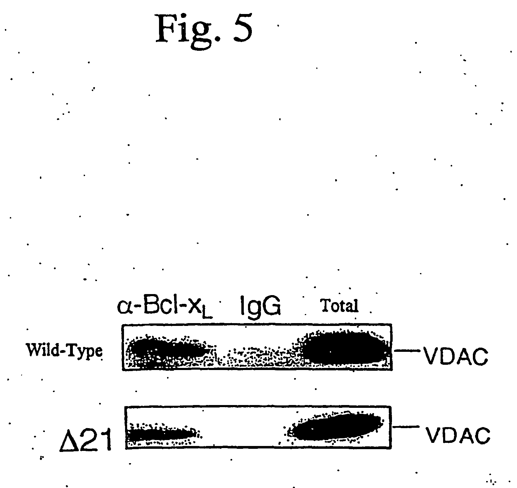

Figure 5 is a diagram showing the results for evaluating the direct interaction between VDAC and Bcl-x_{L} or Δ21. Purified rat liver VDAC (20 µg/ml) was incubated with rBcl-x_{L} protein (20 µg/ml) or rΔ21 protein (20 µg/ml). Each sample was immunoprecipitated with an anti-Bcl-x_{L} antibody, L19 (α-Bcl-x_{L}), and normal rabbit IgG (IgG). Next, each of immunoprecipitated products was assayed by Western blotting using anti-VDAC antibody. "Total" indicates a total amount of VDAC used.

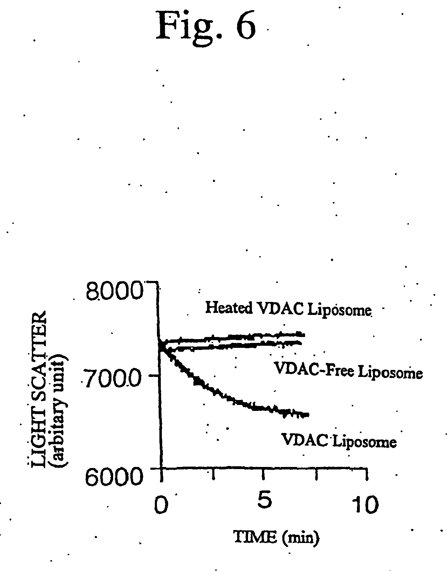

Figure 6 is a diagram showing the results for evaluating sucrose uptake by VDAC liposome. According to the previous report by Shimizu et al. [*Nature,* 399, 483-487 (1999)], VDAC-free liposome, VDAC liposome or heat denatured-VDAC liposome (heated VDAC liposome) was incubated with 50 mM sucrose. Liposome swelling was continuously monitored by the decrease of light scatter using a spectrophotometer at a wavelength of 520 nm.

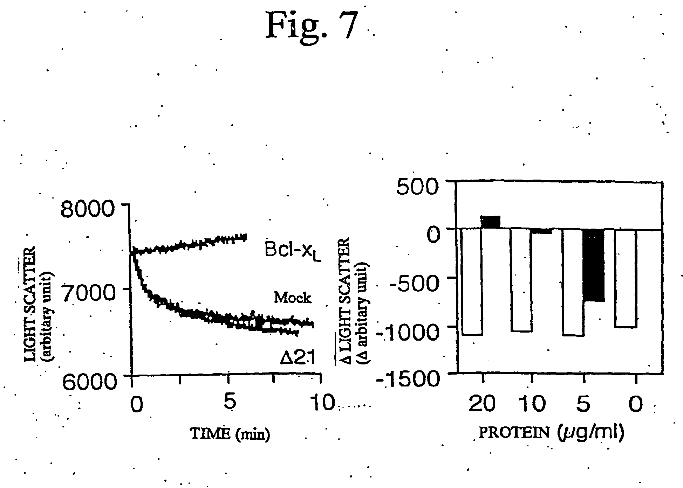

Figure 7 is a diagram showing the results for evaluating the effects of Δ21 on the inhibition of VDAC activity. In the left panel, VDAC liposome was incubated with 50 mM sucrose together with 20 µg/ml rBcl-x_{L}, rΔ21 or mock protein. Light scatter was monitored in the same manner as in Figure 6. The right panel shows the results for evaluating the effect of protein concentration on the inhibition of VDAC activity by Δ21. VDAC liposome of indicated concentrations was incubated with 50 mM sucrose together with rBcl-x_{L} (closed column) or rΔ21 (open column). Differences between light scatter detected at the start time (time 0) and at 10 minutes (Δ light scatter)are indicated.

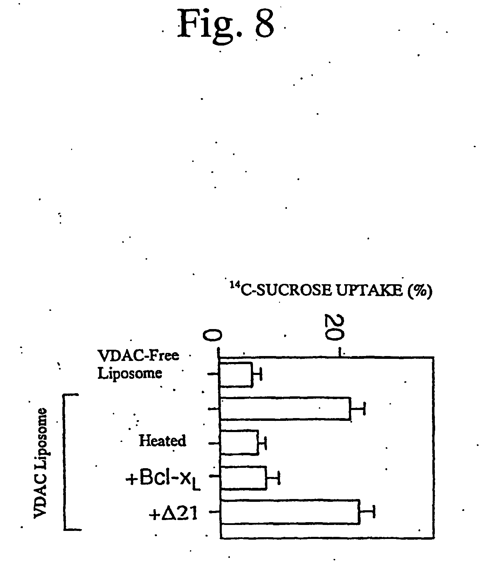

Figure 8 is a diagram showing the results for evaluating the effect of Bcl-x_{L} on ¹⁴C-sucrose uptake into VDAC liposome. According to the previous report by Shimizu et al. *[Nature,* **399**, 483-487 (1999)], VDAC-free liposome, heat denatured-VDAC liposome (heated) or VDAC liposome was incubated for 10 minutes with ¹⁴C-sucrose (2 µCi) in the presence or absence of 20 µg/ml rBcl-x_{L} or rΔ21. After centrifugation and filtration, liposome was collected and dissolved in 2% SDS. Next, ¹⁴C-sucrose radioactivity was counted in the liposome.

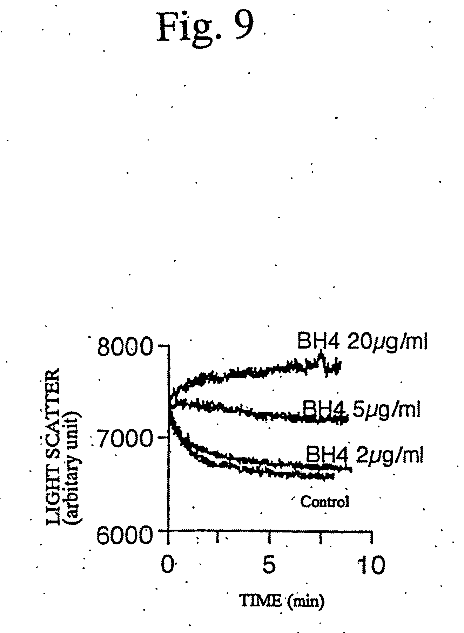

Figure 9 is a diagram showing the results for evaluating the inhibition of VDAC activity by BH4 polypeptide. VDAC liposome was incubated with 50 mM sucrose together with Bcl-x_{L} BH4 polypeptide at indicated concentrations, and light scatter was measured.

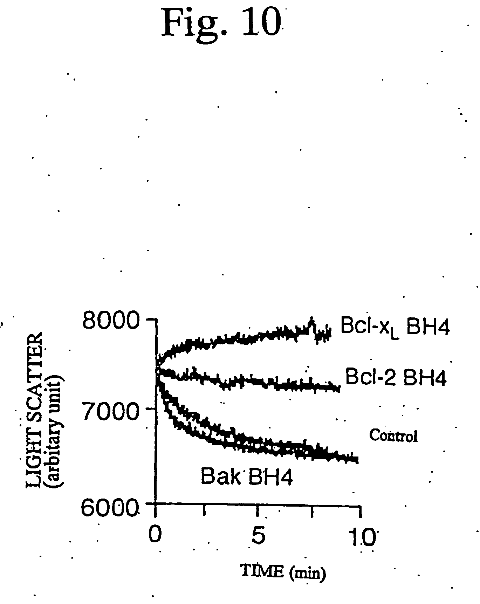

Figure 10 is a diagram showing the results for evaluating the inhibition of VDAC activity by various BH4 polypeptides. VDAC liposome was incubated with sucrose together with 20 µg/ml Bcl-2 BH4 polypeptide, Bcl-x_{L} BH4 polypeptide or Bak-putative BH4 polypeptide, and then light scatter was measured.

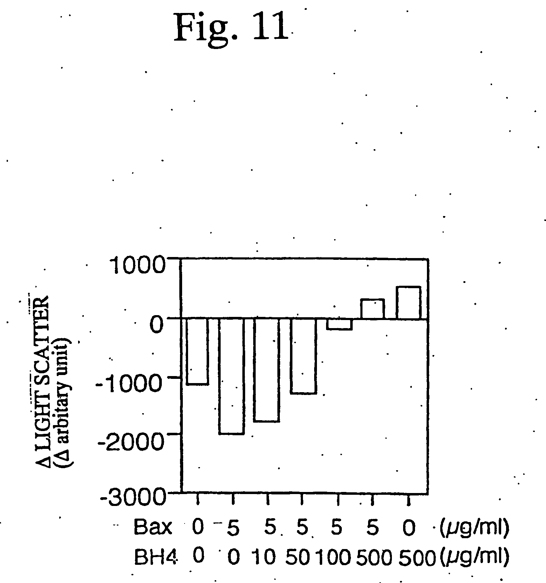

Figure 11 is a diagram showing the results for evaluating the inhibition of Bax-induced enhancement of VDAC activity by Bcl-x_{L} BH4 polypeptide. VDAC liposome was incubated with 50 mM sucrose in the presence or absence of 5 µg/ml rBax and/or Bcl-x_{L} BH4 polypeptide of an indicated concentration, and light scatter was measured. Differences between light scatter detected at the start time (time 0) and at 10 minutes (Δ light scatter) are indicated.

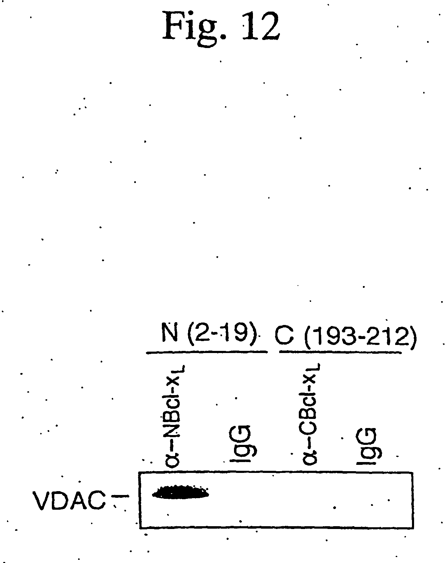

Figure 12 is a diagram showing the results for evaluating the interaction between Bcl-x_{L} N-terminal fragment or C-terminal fragment and VDAC. Mitochondria (1 mg/ml) were incubated for 5 minutes with Bcl-x_{L} N-terminal (amino acid nos: 2-19) polypeptide (20 µg/ml) or Bcl-x_{L} C-terminal (amino acid nos: 193-212) polypeptide (20 µg/ml). Mitochondrial lysate was immunoprecipitated with anti-Bcl-x_{L} antibody (S18) (α-NBcl-x_{L}, an antibody against amino acid nos: 2-19) or L19 antibody (α-CBcl-x_{L}, an antibody against amino acid nos: 193-212). Normal rabbit IgG (IgG) was used as a control. Immunoprecipitated products were analyzed by Western blotting using anti-VDAC antibody.

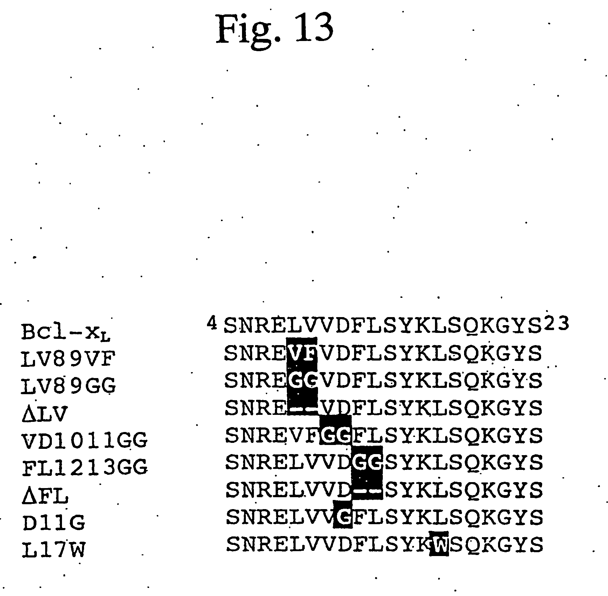

Figure 13 shows the amino acid sequences of mutant Bcl-x_{L} BH4 polypeptides. In the figure, substituted residues are indicated by letters with blackened background.

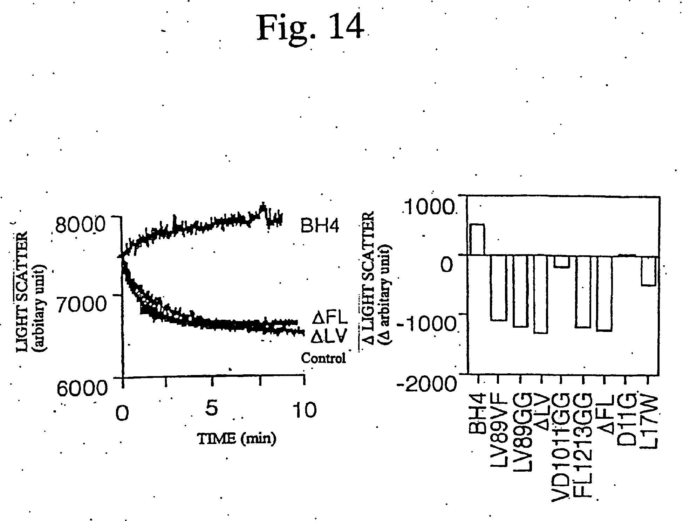

Figure 14 shows the results for evaluating the ability of mutant BH4 polypeptides to inhibit VDAC activity. VDAC liposome was incubated with 20 µg/ml Bcl-x_{L} BH4 polypeptide or mutant polypeptides indicated together with 50 mM sucrose, and light scatter was measured. The left panel shows the results of continuously-monitored light scatter. The right panel shows the difference between light scatter detected at the start time (time 0) and at 10 minutes (Δ light scatter).

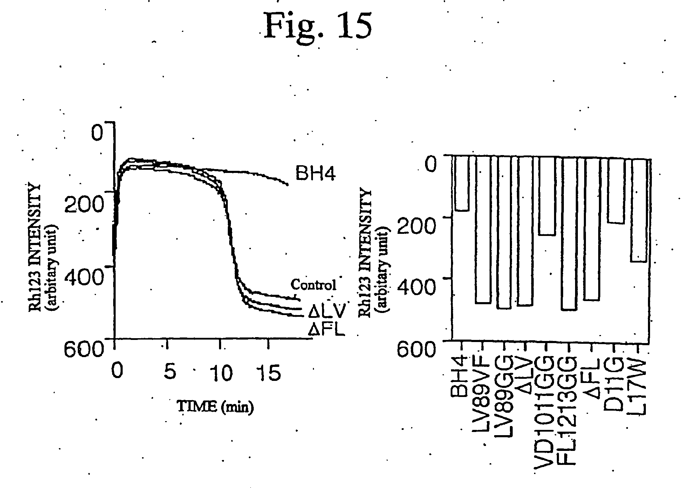

Figure 15 is a diagram showing the results for evaluating the inhibition of Ca²⁺-induced ΔΨ loss from mitochondria by BH4 polypeptide. The left panel shows the results obtained by incubating isolated mitochondria (1 mg/ml) with 20 µg/ml Bcl-x_{L} BH4 polypeptide in the presence of 40 µM Ca²⁺, and then continuously monitoring an intensity for Rhodamine 123. The right panel shows the results obtained by incubating isolated mitochondria (1 mg/ml) with 20 µg/ml Bcl-x_{L} BH4 polypeptide or various mutant polypeptides in the presence of 40 µM Ca²⁺ for 15 minutes and then measuring ΔΨ using an intensity for Rhodamine 123.

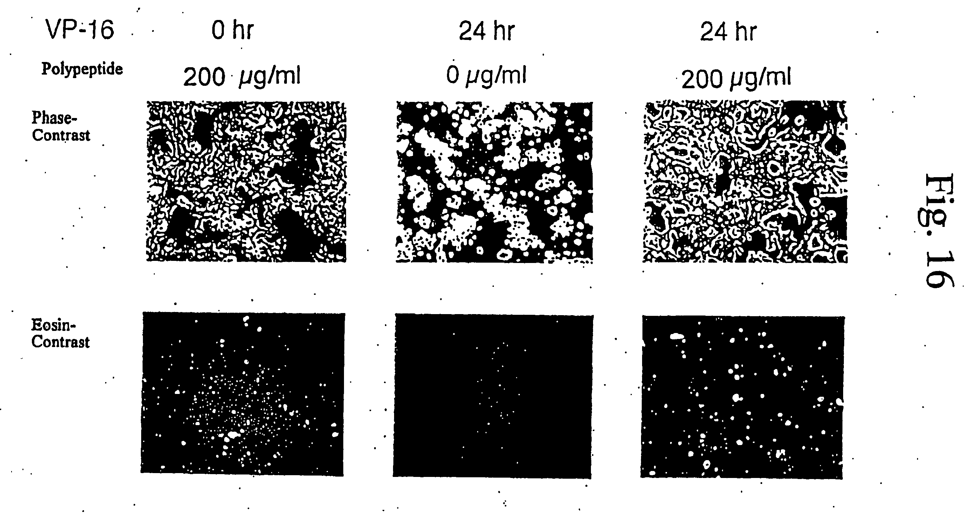

Figure 16 is a diagram showing representative examples of HeLa cells treated with VP16 in the presence or absence of TAT-BH4 polypeptide. HeLa cells were treated with 200 µM VP16 for the indicated periods of time in the presence of TAT-BH4, polypeptide at the indicated concentrations. Next, the morphology of cells (the upper panel) and the accumulation of polypeptide in cells (the lower panel) were observed by a light phase contrast microscope and a fluorescence microscope, respectively.

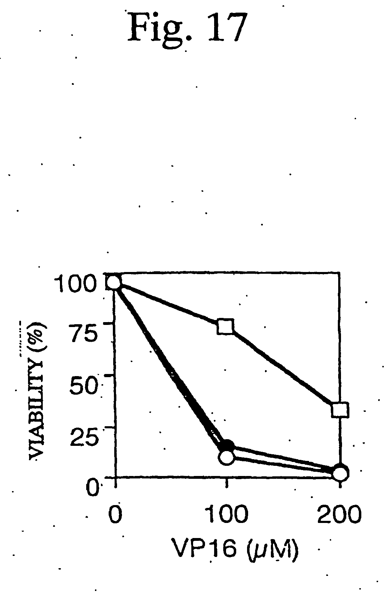

Figure 17 is a diagram showing the results for evaluating the effect of TAT-BH4 polypeptide on VP16-induced apoptosis. Cells were treated with VP16 at the indicated concentrations for 24 hours in the presence of 100 µg/ml TAT-BH4 polypeptide (□) or TAT polypeptide (○) or absence of polypeptide (●) and then stained with Hoechst 33342. Apoptotic cell death was determined by the morphology of nuclei using a fluorescence microscope. Data shown are from one of three independent experiments.

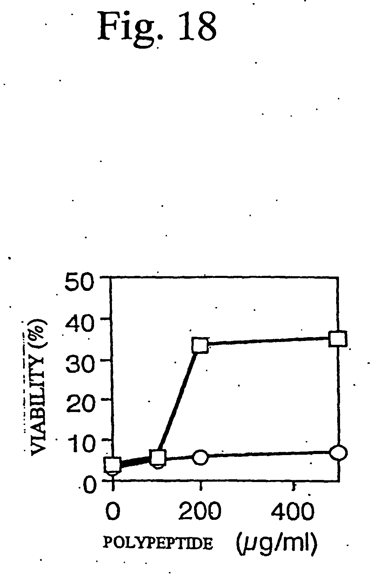

Figure 18 is a diagram showing the results for evaluating the dose-dependent inhibition of VP16-induced apoptosis by TAT-BH4 polypeptide. HeLa cells were treated with 200 µM VP16 for 24 hours in the presence of TAT-BH4 polypeptide (□) or TAT polypeptide (○) at the indicated concentrations. Apoptotic cell death was determined in the same manner as in Figure 17.

### BEST MODE FOR CARRYING OUT THE INVENTION

One of the significant features of the BH4 fusion polypeptide of the present invention resides in that the BH4 fusion polypeptide consists of:
an amino acid sequence of a polypeptide or a derivative sequence thereof capable of exhibiting uptake action into a cell, and
an amino acid sequence selected from the group consisting of:
   (A) an amino acid sequence of SEQ ID NOs:1, 2, 3, 4, 9, 14, 15,16, 17, 18, 19, 20, 21 or Ser 11 to Ser 30 of SEQ ID NO:29; and
   (B) an amino acid sequence having substitution, deletion or insertion of one amino acid residue in the amino acid sequence of SEQ ID NO: 1, 2, 3, 4, 9, 14, 15, 16, 17, 18, 19, 20, 21 or Ser 11 to Ser 30 of SEQ ID NO:29,
wherein the BH4 fusion polypeptide is capable of inhibiting apoptosis.

Since BH4 fusion polypeptide of the present invention includes a minimum unit for inhibiting apoptosis, namely an amino acid sequence selected from the group consisting of:
(A) an amino acid sequence 1, 2, 3, 4, 9, 14, 15, 16, 17, 18, 19, 20, 21 or Ser 11 to Ser 30 of SEQ ID NO:29; and
(B) an amino acid sequence having substitution, deletion or insertion of one amino acid residue in the amino acid sequence of SEQ ID NO:1, 2, 3, 4, 9, 14, 15, 16, 17, 18, 19, 20, 21 or Ser 11 to Ser 30 of SEQ ID NO:29,
the BH4 fusion polypeptide exhibits an excellent characteristic such that the BH4 fusion polypeptide can efficiently inhibit apoptosis. Since the BH4 fusion polypeptide of the present invention comprises a BH4 domain, the mitochondrial ΔΨ loss and the cytochrome c release can be respectively suppressed, so that it is thought that the apoptosis can be inhibited.

Further, since the BH4 fusion polypeptide of the present invention comprises an amino acid sequence of a polypeptide capable of exhibiting uptake action into a cell or a derivative sequence thereof, the BH4 fusion polypeptide can exhibit excellent effects such that the BH4 fusion polypeptide can be more efficiently incorporated into the cell, so that the polypeptide can efficiently inhibit apoptosis.

The term "apoptosis" as used herein refers to a manner of cell death
characterized by changes represented by condensation or fragmentation of nucleus of cell, condensation or fragmentation of the cell itself, and fragmentation of chromosomal DNA of the cell into nucleosome units (about 180 bp), and includes those events caused by pathological factors and physiological factors (for instance, expression of physiological events such as immunization, hormone or development).

The term "anti-apoptotic Bcl-2 family protein" means Bcl-2 protein and those possessing an action of inhibiting apoptosis among proteins similar to the Bcl-2 protein. An activity of inhibiting apoptosis will herein be referred to as "anti-apoptotic activity," and an activity for promoting apoptosis will be referred to as "pro-apoptotic activity."

The inhibition and promotion of apoptosis can be evaluated by, for instance, the following method, without being limited thereto. The method comprises treating cells with VP16 for 24 hours, thereafter staining the cells with 1 µM Hoechst 33342, and then calculating an extent of apoptosis as the ratio of cells exhibiting nuclear fragmentation to the total cells.

In addition, the anti-apoptotic activity can be evaluated by the reduction of the mitochondrial ΔΨ and the inhibition of the cytochrome c release.

ΔΨ can be evaluated by determining uptake of Rhodamine 123 (Rh123) as previously described in, for instance, Shimizu et al. [*Oncogene,* **13,** 21-29 (1996)].

The cytochrome *c* release can be evaluated by preparing mitochondria, centrifuging the mitochondria to obtain mitochondria pellets and supernatant, resuspending the resulting pellets in an RIPA buffer, and subjecting the resulting suspension of mitochondria and the supernatant to Western blotting with an anti-cytochrome *c* antibody.

The above-mentioned anti-apoptotic Bcl-2 family proteins have sequence homologies in Bcl-2 homology (BH)1, BH2, BH3 and BH4 domains. The above-mentioned BH1 and BH2 domains, and probably BH3 domain are crucial in the dimerization with any of the proteins belonging to the pro-apoptotic factor family protein as determined by mutatiorially structural analysis, by which it is thought that pro-apoptotic activity is inhibited.

The amino acid sequence of SEQ ID NO: 1 is a sequence of BH4 domain which is commonly conserved only among the proteins belonging to anti-apoptotic Bcl-2 family protein. In the BH4 domain, it is thought that α-helix is crucial for action for inhibiting apoptosis by a polypeptide comprising the BH4 domain, and that especially well conserved amino acid residues (amino acid nos: 8 and 9 in SEQ ID NO: 1) play an important role in the formation of the α-helix structure. Further, it is deduced that the amino acid residues of amino acid nos: 5, 6, 9, 10 and 14 in SEQ ID NO: 1 are aligned on the binding surface of α-helix of the above-mentioned BH4 domain to another protein.

Therefore, the BH4 domain of the above-mentioned anti-apoptotic Bcl-2 family protein may have an amino acid sequence having substitution, deletion or insertion of one amino acid residue in the amino acid sequence of SEQ ID NO: 1, 2, 3, 4, 9, 14, 15, 16, 17, 18, 19, 20, 21 or Ser 11 to Ser 30 of SEQ ID NO:29. A polypeptide having the amino acid sequence comprising the mutation is a polypeptide capable of exhibiting an anti-apoptotic activity. The above-mentioned anti-apoptotic activity can be examined by evaluating the reduction of the mitochondrial ΔΨ and the inhibition of the cytochrome c release according to the methods described above.

Here, "amino acid sequence having substitution, deletion or insertion of at least one amino acid residue" can be arbitrarily prepared in accordance with the desired sequence when a peptide is synthesized by a known method.

A BH4 domain of an anti-apoptotic Bcl-2 family protein may have an amino acid sequence having at least 50%, preferably 70% or higher, and more preferably 90% or higher sequence identity to the amino acid sequence of SEQ ID NO: 1, as long as the BH4 domain has a sequence in which amino acid nos: 5, 6, 8, 9, 10 and 14 in the above-mentioned amino acid sequence of SEQ ID NO: 1 are conserved.

The above polypeptide having the sequence identity is a polypeptide capable of exhibiting anti-apoptotic activity. The above-mentioned anti-apoptotic activity can be examined by evaluating the reduction of the mitochondrial ΔΨ and the inhibition of cytochrome c release according to the methods described above.

The term "sequence identity" as used herein refers to the identity of amino acid residues between two amino acid sequences, namely between the amino acid sequence of SEQ ID NO: 1 and an amino acid sequence to be compared thereto. The above-mentioned "sequence identity" can be determined by comparing two amino acid sequences which are optimally aligned with each other over the region of the amino acid sequence to be compared. Here, the amino acid sequence to be compared may have addition or deletion (for instance, gap, overhang or the like), as compared to the amino acid sequence of SEQ ID NO: 1 given herein.

The numerical value (percentage) of the sequence identity can be calculated by determining identical amino acid residues which are present in both of the sequences to determine the numbers of matched sites, thereafter dividing the above-mentioned number of the matched site by a total number of the amino acid residues present within the region of the sequence to be compared, and multiplying the resulting numerical value by 100. An algorithm for obtaining optimal alignment and homology includes, for instance, BLAST and the like. Concretely, the optimal alignment and homology can be obtained by gene analysis software GENETYX-SV/RG Ver. 4.01 (manufactured by Software Development), gene analysis program BLAST [internet address: http://www.ncbi.nlm.nih.gov/BLAST; reference: Altschul, S.F et al., *J. Mol*. *Biol.,* **215**, 403-410, (1990), Altschul, S.F. et al., *Nucl. Acid Res.,* **25**, 3389-3402, (1997)], or the like.

In addition, a BH4 domain of an anti-apoptotic Bcl-2 family protein may have an amino acid sequence having at least 50%, preferably 70% or higher, and more preferably 90% or higher homology to the amino acid sequence of SEQ ID NO: 1, as long as the polypeptide having the domain exhibits the anti-apoptotic activity, namely the reduction of the mitochondrial ΔΨ and the inhibition of the cytochrome c release determined according to the methods described above. Here, the BH4 domain of the above-mentioned anti-apoptotic Bcl-2 family protein may have an amino acid sequence having conservative substitution. The above-mentioned "conservative substitution" encompasses substitution with an amino acid possessing similar properties (i.e., hydrophobicity, electric charge, pK, features in three-dimensional structure); and substitution with an amino acid which would cause any changes in the three-dimensional structure and folding structure of the polypeptide only to an extent so that the physiological activity inherently owned by the polypeptide is maintained. The conservative substitution includes, for instance, the followings: substitutions within the groups of: 1) glycine and alanine; 2) valine, isoleucine and leucine; 3) aspartic acid, glutamic acid, aspartic acid and glutamine; 4) serine and threonine; 5) lysine and arginine; or 6) phenylalanine and tyrosine.

Concrete examples of the BH4 domain include (a) Bcl-2 BH4 domain (SEQ ID NO: 2), (b) Bcl-x_{L} BH4 domain (SEQ ID NO: 3), (c) Bcl-w BH4 domain (SEQ ID NO: 4), (d) C. *elegans* Ced-9 BH4 domain and the like. In the present invention, the Bcl-2 BH4 domain (SEQ ID NO: 2) and the Bcl-x_{L} BH4 domain (SEQ ID NO: 3) are preferable. In addition, the BH4 domain may be an amino acid sequence having substitution, deletion or insertion of one amino acid residue in any one of the above-mentioned amino acid sequences (a) to (d). Also described are BH4 domains having an amino acid sequence having at least 50%, preferably 70% or higher, and more preferably 90% or higher sequence identity to any one of the above-mentioned amino acid sequences (a) to (d), as long as the domain exhibits the reduction of the mitochondrial ΔΨ and the inhibition of the cytochrome c release determined according to the methods described above.

The polypeptide used in the BH4 fusion polypeptide of the present invention, capable of exhibiting uptake action into a cell includes, for instance, TAT from human immunodeficiency virus (HIV), p27 from HTLV-I, art/trs from HIV, VP22 from HSV-1, Antp from *Drosophila* and the like.

The amino acid sequence of the polypeptide capable of exhibiting uptake action into a cell includes, for instance:
(i) a sequence comprising at least amino acid nos: 49 to 57 of the amino acid sequence of SEQ ID NO: 5;
(ii) a sequence comprising at least amino acid nos: 1 to 13 of the amino acid sequence of SEQ ID NO: 24;
(iii) a sequence comprising at least amino acid nos: 1 to 16 of the amino acid sequence of SEQ ID NO: 25;
(iv) a sequence comprising at least amino acid nos: 1 to 301 of the amino acid sequence of SEQ ID NO: 26;
(v) a sequence comprising at least amino acid nos: 1 to 16 of the amino acid sequence of SEQ ID NO: 27;
and the like. Among them, an amino acid sequence comprising a region consisting of at least amino acid nos: 49 to 57 (SEQ ID NO: 6) of TAT sequence (SEQ ID NO: 5) from human immunodeficiency virus is preferable.

The above-mentioned "derivative sequence of the amino acid sequence of a polypeptide capable of exhibiting uptake action into a cell" includes, for instance,
(I) an amino acid sequence having a chemically modified group of at least one residue in the above-mentioned amino acid sequence of the polypeptide capable of exhibiting uptake action into a cell, or
(II) an amino acid sequence having substitution or insertion of at least one amino acid residue in the above-mentioned amino acid sequence of the polypeptide capable of exhibiting uptake action into a cell,
wherein the resulting polypeptide is capable of exhibiting uptake action into a cell.

The uptake action into a cell can be examined on a microscope by immunologically staining with an antibody against the peptide or introducing a fluorescent-labeling into the peptide.

The chemically modified group includes functional groups exhibiting fluorescence, functional groups not involved in the formation of the three-dimensional structure of polypeptide, and the like, as long as the polypeptides comprising an amino acid sequence having the chemically-modified amino acid residue are capable of exhibiting uptake action into a cell. The term "chemically modified group" as referred to herein may be an amino acid residue (for instance, β-alanine or the like) other than the amino acid residues constituting a naturally-occurring "polypeptide capable of exhibiting uptake action into a cell."

The functional group exhibiting fluorescence includes eosin, fluorescein isothiocyanate (FITC), and the like.

The functional group not involved in the formation of the three-dimensional structure of the polypeptide includes spacer groups represented by β-alanine residue or the like. It is preferable that the functional-group is present at the terminal at which the "polypeptide capable of exhibiting uptake action into a cell" is fused to the "BH4 domain of anti-apoptotic Bcl-2 family protein." By introducing the functional group, each of the "polypeptide capable of exhibiting uptake action into a cell" and the "BH4 domain of anti-apoptotic Bcl-2 family protein" can maintain their inherent three-dimensional structures, so that their functions can be sufficiently exhibited.

It is preferable that the BH4 fusion polypeptide of the present invention is a polypeptide consisting of an amino acid sequence of the region consisting of at least amino acid nos: 49 to 57 (SEQ ID NO: 6) of TAT sequence (SEQ ID NO: 5) from human immunodeficiency virus, and (a) the sequence of the BH4 domain (SEQ ID NO: 2) of Bcl-2 protein, (b) an amino acid sequence of the BH4 domain (SEQ ID NO: 3) of Bcl-x_{L} protein, (c) an amino acid sequence of the BH4 domain (SEQ ID NO: 4) of Bcl-w protein, or (d) a BH4 domainof *C*. *elegans* Ced-9.

It is preferable that the BH4 fusion polypeptide of the present invention is concretely a polypeptide comprising one amino acid sequence selected from the group consisting of the amino acid sequences of SEQ ID NOs: 7, 8, 28, 29, 30, 31, 32 and 33.

Cys (TAT)-β-Ala-BH4 peptide, which is a BH4 fusion peptide of the present invention, can be synthesized by Fmoc method using Fmoc-linked amide resin in ABI433A automated synthesizer. The resulting protected peptide resin is treated with a reagent containing trifluoroacetic acid, and free peptide is then purified by reverse phase HPLC. Next, eosin-5-iodoacetamide dissolved in DMF was mixed with the above-mentioned purified peptide dissolved in 50 mM phosphate buffer (pH 7.7), and the mixture was allowed to react at room temperature for 1 hour. After the reaction, the peptide is purified again by reverse phase HPLC, and collected as a single peak. The molecular weight of the purified sample is determined by MALDI-TOF MS.

According to the BH4 fusion polypeptide of the present invention, for instance, as the apoptosis-inhibitory ability in HeLa cell, there can be expected to have an inhibition ratio of at least 50%, preferably 70% or higher, and more preferably 90% or higher, as compared to those in the absence of the BH4 fusion polypeptide.

Since the BH4 fusion polypeptide of the present invention exhibits anti-apoptotic activity, the BH4 fusion polypeptide can be used as an active ingredient in an apoptosis-inhibitor. The apoptosis-inhibitor is also encompassed in the present invention.

One of the features in the apoptosis-inhibitor of the present invention resides in that the apoptosis-inhibitor comprises the above-mentioned BH4 fusion polypeptide.

Since the apoptosis-inhibitor of the present invention comprises the BH4 fusion polypeptide exhibiting an anti-apoptotic activity, there can be expected therapeutic effects for AIDS, neurodegenerative disorders (such as Alzheimer's disease, Parkinson's disease, amyotrophic lateral sclerosis, retinitis pigmentosa or cerebellar degeneration), osteomyelodysplasia (aplastic anemia and the like), ischemic diseases (myocardial infarction, brain infarction, apoplectic stroke, ischemic reperfusion disorder and the like), fulminant hepatitis, hepatic failures caused by addictions such as an alcoholism, infectious multiple organ failure, diabetes and the like. The present invention also encompasses uses therein the above-mentioned apoptosis-inhibitor is administered to a patient with the above-mentioned diseases to inhibit apoptosis, thereby treating those diseases. The treatment is, among all, suitable for treatment of ischemic diseases, particularly myocardial infarction and brain infarction, and especially myocardial infarction. Alternatively, by adding the apoptosis-inhibitor to a stock solution for preserving an organ to be used for implantation, the organ can be stored in a more preferable state until implantation.

The apoptosis-inhibitor of the present invention may comprise the BH4 fusion polypeptide in any modified form so that the uptake of the above-mentioned BH4 fusion polypeptide into a particular cell is facilitated. In addition, the apoptosis-inhibitor of the present invention may contain various aids which are usually employed within a range so that anti-apoptotic activity can be exhibited.

When the apoptosis-inhibitor is administered, the administration form, the administration method and the dose can be suitably set, depending upon age, weight and conditions of an individual to be administered.

Further, according to the present invention, there is provided use of BH4 fusion polypeptide for the manufacture of a prophylactic or therapeutic agent for the above-mentioned diseases; notably ischemic diseases, particularly myocardial infarction and brain infarction, and especially myocardial infarction.

The present invention will be concretely described by means of Examples and the like, without intending to limit the scope of the present invention thereto.

### Reagents

As an anti-pigeon cytochrome *c* monoclonal antibody (7H8.2C12) cross-reacting with human cytochrome *c* and rat cytochrome c, one kindly provided by Dr. E. Margoliash (University of Illinois, Illinois) was used. As an anti-human VDAC (porin) monoclonal antibody (31HL) cross-reacting with rat VDAC, one obtained from Calbiochem was used. As anti-human Bcl-x_{L} polyclonal antibodies [L19 specific to the region of amino acid nos: 193 to 212 of human Bcl-x_{L} amino acid sequence (SEQ ID NO: 10), and S18 specific to the region of amino acid nos: 2-19 of the amino acid sequence], those obtained from Santa Cruz Biotechnology were used. As hydroxyapatite and celite, those obtained from Bio-Rad and Roth, respectively, were used. As diisopropylcarbodiimide/1-hydroxybenzotriazole-activated Fmoc-protected amino acid, one obtained from Genzyme-Syena was used. As other reagents and the like, those reagents manufactured by Wako Pure Chemical Industries, Ltd. were used unless otherwise indicated.

### Reference Example 1: DNA Transfection and Apoptosis Assay

Human bcl-x_{L} mutant DNAs were prepared by polymerase chain reaction (PCR) using a proofreading *Pfu* DNA polymerase (manufactured by Stratagene), human B cell-derived cDNA library (manufactured by Clontech) as a template and oligonucleotides of each of SEQ ID NO: 11 and SEQ ID NO: 12 as primers. The sequence of the above-mentioned human bcl-x_{L} mutant. DNA is shown in SEQ ID NO: 13 of Sequence Listing. Each of the resulting amplified fragments was subcloned into pUC-GAGGS expression vector [Shimizu, et al., *Oncogene,* **13***,* 21-29 (1996)].

Using lipofectamine, HeLa cells, a human cervical carcinoma cell line, were transfected for 24 hours with the expression plasmid (0.1 µg) for human Bcl-x_{L} or mutant thereof together with the expression construct pEGFP-N1 (manufactured by Clontech) of green fluorescence protein (hereinafter referred to as GFP) (0.1 µg) for monitoring DNA transfection.

The resulting transfected cells were treated with VP16 for 24 hours. Thereafter, the cells were stained with 1 µM Hoechst 33342, and the extent of apoptosis was then calculated as the percentage of GFP-positive cells exhibiting nuclear fragmentation to all the positive cells.

In addition, in the case of decay-accelerating factor (DAF) expression, HeLa cells were transfected for 24 hours with the expression plasmids (0.1 µg) for human Bcl-x_{L} or mutant derivative thereof together with 0.1 µg of DAF expression construct (pUC-CAGGS-DAF). After 24 hours, the resulting cells were stained with anti-human DAF antibody and FITC-conjugated anti-mouse IgG for 1 hour. Thereafter, DAF-positive cells were sorted using a cell sorter [trade name: FACS-Vantage manufactured by Becton-Dickinson], and expression of DAF and Bcl-x_{L} was assessed by a flow cytometer. The DAF-positive cells sorted as described above were treated with 200 µM VP16 for 24 hours. Thereafter, the cells were treated with 10 µM digitonin at 37°C for 10 minutes, and supernatant and pellets were separated by centrifugation. The amount of cytochrome c was estimated by Western blot analysis using anti-pigeon cytochrome c antibody.

### Reference Example 2: Measurement of Mitochondrial Biochemical Parameters (ΔΨ and Cytochrome c Release)

Rat liver mitochondria (1 mg protein/ml) isolated in accordance with a report made by Shimizu et al. *[Oncogene,* **13***,* 21-29(1996)] were incubated at 25°C in a medium containing 0.3 M mannitol, 10 mM HEPES/K⁺ (pH 7.4), 0.1% fatty acid-free BSA, 1 mM potassium phosphate, 40 µM CaCl₂ and 4.2 mM succinate for energization in the presence or absence of recombinant proteins.

ΔΨ was assessed by measuring the uptake of Rhodamine 123 (Rh123) as previously described by Shimizu et al. [*Oncogene,* **13**, 21-29(1996)].

The cytochrome c release was evaluated by centrifuging the above-mentioned mitochondria, re-suspending the resulting pellets in an RIPA buffer to give mitochondria suspension, and subjecting the mitochondria suspension and supernatant to Western blot analysis using anti-cytochrome c antibodies.

### Reference Example 3: Immunoprecipitation and Western Blot Analysis

Purified VDAC (20 µg/ml) was incubated with recombinant Bcl-x_{L} (rBcl-x_{L}) and recombinant Δ21 (rΔ21), and the resulting mixture was immunoprecipitated with anti-Bcl-x_{L} (L19) antibody and normal rabbit IgG. In order to detect interactions between polypeptide and VDAC, isolated mitochondria (1 mg) were incubated with 20 µg of Bcl-x_{L} N-terminal polypeptide or Bcl-x_{L} C-terminal polypeptide for 5 minutes. Thereafter, the resulting mitochondria were pelleted, washed, and suspended in a lysis buffer (10 mM HEPES, pH 7.4, 142.5 mM KCl, 5 mM MgCl₂, 1 mM EGTA and 0.5% NP40) in the presence of proteinase inhibitors (0.1 mM p-APMSF, 10 µg/ml aprotinin, 1 µg/ml chymostatin, 1 µg/ml leupeptin, 1 µ/ml antipain, 1 µg/ml pepstatin), followed by ultrasonic disruption. Immunoprecipitation was carried out with anti-Bcl-x polyclonal antibodies corresponding to the polypeptides used. Co-immunoprecipitation of VDAC was detected by Western blotting using an anti-VDAC antibody.

### Reference Example 4: Reconstitution of VDAC in Liposomes

Purified VDAC was reconstituted in small unilamellar vesicles by the ultrasonic freeze-thaw procedure described in a report made by Shimizu et al. [*Nature,* **399***,* 483-487 (1999)]. Sucrose uptake experiment was performed by assessing liposomal swelling.

Liposomes produced at pH 5.2 were incubated at 25°C in 1 ml of liposome buffer together with rBcl-x_{L}, rΔ21, rBax or a polypeptide for 3 minutes. Here, acidic pH was a required condition for efficient uptake of rBcl-x_{L} and rBax into the liposomes. Thereafter, sucrose was added up to a concentration of 50 mM to the mixture, and the liposomal swelling was assessed by the decrease in light scatter at a wavelength of 520 nm using a spectrophotometer (F-4500, manufactured by Hitachi, Ltd.). Sucrose uptake was also estimated from ¹⁴C-sucrose uptake as described in the report made by Shimizu et al. *[Nature,* **399**, 483-487(1999)].

### Example 1: Purification of Human Bcl-x_{L} and Human Bcl-x_{L}Δ21

Human Bcl-xL and Bcl-x_{L}Δ21 (lacking the N-terminus 21 amino acids residues of Bcl-x_{L}) were expressed as glutathione-S-transferase (GST) fusion proteins in *Escherichia coli* strain DH5α, and the resulting fusion proteins were purified on a glutathione-sepharose column. Next, human Bcl-x_{L} or Bcl-x_{L}Δ21, and GST were separated by cleavage with thrombin. In addition, human Bax was obtained in accordance with literature by Narita et al. *[Proc. Natl. Acad. Sci., USA,* **95,** 14681-14686 (1988)]. The resulting purified proteins were dissolved in a buffer [composition: 20 mM Tris-HCl (pH 7.4), 2 mM MgCl₂, 1 mM dithiothreitol]. GST proteins derived from a vector carrying no insert were prepared, and used as mock control proteins. Rat liver VDAC was purified in accordance with a report made by Shimizu et al. [*Nature,* **399,** 483-487 (1999)]. The resulting VDAC showed a single band on SDS-polyacrylamide electrophoresis.

### Example 2: Synthesis of Polypeptides

Polypeptides were synthesized with a Model 396 Multiple Polypeptide synthesizer [manufactured by Advanced Chemtech], using diisopropylcarbodiimide/1-hydroxybenzotriazole-activated, Fmoc-protected amino acids.

Synthesized polypeptides were as follows:
Synthesized Polypeptides:
   Human Bcl-x_{L} BH4 (amino acid nos: 4 to 23 of human Bcl-x_{L}):
      SNRELVVDFLSYKLSQKGYS (SEQ ID NO: 3);
   Mutants of Human Bcl-x_{L} BH4 (amino acid nos: 4 to 23 of human Bcl-x_{L} BH4) shown in Figure 14 (SEQ ID NOs: 14 to 21);
   Human Bcl-2 BH4 (amino acid nos: 7 to 30 of human Bcl-2 BH4):
      TGYDNREIVMKYIHYKLSQRGYEW (SEQ ID NO: 2); and
   Human Bak BH4 (amino acid nos: 27 to 50 of human Bak BH4)
      VAQDTEEVFRSYVFYRHQQEQEAE (SEQ ID NO: 22).

It was confirmed that the purity for each of the above-mentioned polypeptides was exceeding 95% according to matrix-assisted laser desorption ionization-time of flight mass spectrometry (MALDI-TOF MS).

The N-terminal (amino acid nos: 2 to 19) polypeptide of Bcl-x_{L} and the C-terminal (amino acid nos: 193 to 212) polypeptide of Bcl-x_{L} were purchased from Santa Cruz Biotechnology.

There was synthesized a polypeptide [eosin-C-RKKRRQRRR (SEQ ID NO: 23)], resulting from introduction of an eosin-conjugated cystein residue into an N-terminal of a polypeptide TAT-PTD, corresponding to the protein transduction domain (PTD) (amino acid nos: 49 to 57) of HIV TAT (SEQ ID NO: 5). Thereafter, TAT-PTD-BH4 polypeptide (TAT-BH4 polypeptide) was prepared by linking eosin-conjugated TAT-PTD polypeptide to human Bcl-x_{L} BH4 polypeptide via β-alanine residue.

It was confirmed that the resulting TAT-BH4 polypeptide had a purity of 90% or higher by monitoring fluorescence derived from the protein (absorption at 280 nm) using reverse-phase HPLC.

### Example 3: Importance of BH4 Domain in Inhibition of Apoptotic Mitochondrial Changes by Bcl-x_{L}.

Bcl-2/Bcl-x_{L} inhibits apoptosis by blocking apoptotic mitochondrial changes. Therefore, there was evaluated whether or not the BH4 domain is required for Bcl-2/Bcl-x_{L} to inhibit apoptotic mitochondrial changes.

HeLa cells were transfected in accordance with Reference Example 1 with DNA corresponding to Bcl-x_{L} or its BH4 deletion mutant (Δ21: lacking amino acid residues of 2 to 21-positions of Bcl-x_{L}), and apoptotic assay was performed. The HeLa cells were transiently transfected with an expression construct for Bcl-x_{L} or Δ21 or an empty vector together with an expression construct for DAF. Cell surface expression of DAF was used to identify cells transfected with the above-mentioned DNAs, and these cells were then collected using a flow cytometer.

As a result, as shown in Figure 1, the transfected cells expressing Bcl-x_{L} having the BH4 domain show resistance against apoptotic induction by 100 µM or 200 µM VP16, whereas the transfected cells expressing Δ21 lacking the BH4 domain and the cells transfected with the empty vector show no resistance against apoptotic induction by 100 µM VP16.

In addition, as shown in Figure 2, DAF-positive cells having the purity of exceeding 93% was obtained. In addition, almost all of the resulting DAF-positive cells expressed Bcl-x_{L} or Δ21 at a comparative level.

Thereafter, the collected cells were treated with VP16 to induce apoptosis, and cytochrome c release was tested. As a result, as shown in Figure 3, overexpression of Bcl-x_{L} inhibited cytochrome c release from mitochondria after VP16 treatment, whereas overexpression of Δ21 did not inhibit cytochrome c release, indicating that the BH4 domain was required to inhibit apoptotic cytochrome c release. Also, consistent with these results, as shown Figure 4, when isolated mitochondria were used, recombinant Bcl-x_{L} (rBcl-x_{L}) inhibited both Ca²⁺-induced mitochondrial ΔΨ loss and cytochrome c release, whereas recombinant Δ21 (rΔ21) did not virtually show inhibitory activity for each of mitochondrial ΔΨ loss or cytochrome c release. In addition, similar results were obtained when mitochondria were subjected to other apoptotic stimuli such as atractyloside or hydrogen peroxide.

### Example 4: Role of BH4 Domain in VDAC Regulation

It has been known that Bcl-x_{L} directly binds to VDAC and inhibits the activity of this channel, thereby leading to inhibition of apoptotic cytochrome c release and ΔΨ loss. In addition, interaction between Bcl-x_{L} and VDAC has also been detected in cells as described in a report made by Shimizu et al. *[Nature,* **399**, 483-487 (1999)]. Therefore, the functional role of the BH4 domain in VDAC regulation was examined.

First, co-immunoprecipitation analysis was performed in accordance with Reference Example 3, and the effect of the deletion of the BH4 domain on the interaction between Bcl-x_{L} and VDAC was examined. As a result, as shown in Figure 5, the co-immunoprecipitation analysis revealed that Bcl-x_{L} and VDAC interacted with VDAC to a similar level, suggesting that the BH4 domain was not important for binding between Bcl-x_{L} and VDAC.

Next, the functional role of the BH4 domain in the regulation of VDAC activity was examined. VDAC activity was measured by assessing sucrose uptake into VDAC-containing liposomes, using the two procedures: a method with radiolabeled sucrose described in a report made by Shimizu et al. [*Nature,* **399**, 483-487 (1999)], and a method for measuring a sucrose uptake-dependent liposomal swelling that was monitored by decrease in light scatter. The results are shown in Figure 6. Since sucrose-uptake-dependent liposomal swelling was confirmed by microscopic observation or flow cytometric analysis, it is thought that the liposomal swelling is based on the rapid influx of sucrose and water through large VDAC pores which overwhelmed osmosis-dependent efflux of water. VDAC-liposomes, as shown in Figure 6, developed swelling in the presence of sucrose, whereas VDAC-free liposomes and denatured VDAC-liposomes did not show swelling, indicating that sucrose uptake was mediated by VDAC.

As shown in Figure 7, addition of rBcl-x_{L} to VDAC-liposomes inhibited VDAC-mediated sucrose uptake, whereas addition of rΔ21 to VDAC-liposomes did not affect VDAC-mediated sucrose uptake, indicating that BH4 was essential for inhibition of VDAC by Bcl-x_{L}. In addition, as shown in Figure 8, similar results were obtained when VDAC activity was directly measured by assessing the influx of radiolabeled sucrose.

It is suggested from these results that the BH4 is required for Bcl-x_{L} to inhibit VDAC activity, even though the BH4 domain does not significantly affect the binding to VDAC.

### Example 5: Involvement of BH4 Domain of Bcl-x_{L} in Inhibition of VDAC Activity

In order to elucidate the role of the BH4 domain in inhibiting VDAC activity, a polypeptide corresponding to the domain of Bcl-x_{L} was added to VDAC liposomes to evaluate VDAC activity. As a result, as shown in Figure 9, the BH4 polypeptide inhibited the VDAC activity in a concentration-dependent manner.

The increase in light scatter detected in the presence of 20 µg/ml BH4 polypeptide (shown in Figure 9) was due to the osmosis-dependent shrinkage of VDAC liposomes after complete closure of VDAC by the BH4 polypeptide. As shown in Figure 10, the BH4 polypeptide derived from Bcl-2 also inhibited VDAC activity, whereas a corresponding polypeptide derived from Bak was inactive. The B_{H4} polypeptides derived from Bcl-2 and Bcl-x_{L} did not show any significant effect on VDAC-free liposomes. In accordance with the report made by Shimizu et al. [*Nature,* **399**, 483-487 (1999)], Bax and Bak enhanced the activity of VDAC, and this effect was antagonized by Bcl-x_{L}. As shown in Figure 11, the BH4 polypeptide inhibited the rBax-induced enhancement of VDAC activity in a dose-dependent manner, whereas the BH4 polypeptide derived from Bcl-x_{L} did not bind to Bax.

It is suggested from these results that the antagonistic influence of rBcl-x_{L} and Bax on VDAC is not due to the formation of heterodimers, but due to their independent actions. In addition, although VDAC liposome experiments have generally been carried out at an acidic pH that facilitated the insertion of rBcl-x_{L} into membranes [Shimizu et al., *Nature,* **399**, 483-487 (1999)], virtually identical results were also obtained when the polypeptides were used at a neutral pH.

As shown in Figure 12, consistent with the inhibition of VDAC by the BH4 polypeptide of Bcl-x_{L}, the interaction between the N-terminal fragment (amino acid nos: 2 to 9) of Bcl-x_{L} and VDAC was detected, although to a lesser extent, as compared to that of rBcl-x_{L}. Since Bcl-x_{L} mainly binds to VDAC at some regions other than BH4, other regions may enhance the activity of BH4, probably by increasing affinity and/or accessibility to VDAC. It is suggested from these results that the BH4 domain of anti-apoptotic Bcl-2 family protein was sufficient to inhibit VDAC.

It has been reported that some of the conserved residues in the BH4 domain, such as L⁸V⁹ or F¹², in Bcl-x_{L} are crucial for the anti-apoptotic activity of Bcl-2/Bcl-x_{L}. In order to assess the role of the above-mentioned conserved residues in the inhibition of VDAC by the BH4 domain, several kinds of BH4 mutant polypeptides shown in Figure 13 were prepared, and their inhibitory effect on VDAC was examined. The results are shown in Figure 14.

As shown in Figure 14, L⁸V⁹ mutant polypeptides (LV89VF, LV89GG and ΔLV) and F¹²L¹³ mutant polypeptides (FL1213GG and ΔFL) did not inhibit VDAC. Further, polypeptides with one amino acid substitution at D¹¹ (D11G) and one amino acid substitution at L¹⁷ (L17W), respectively, showed VDAC inhibitory activities of about 70% and about one-half, as compared to VDAC inhibition of the normal BH₄ polypeptide, which was consistent with the decrease in anti-apoptotic activity as described in a report made by Huang, D.C., et al. [Huang, D.C., et al., *EMBO J.,* **117,** 1029-1039 (1998)]. It is suggested in these results that the anti-apoptotic function of Bcl-x_{L} was mediated by its inhibition of VDAC activity.

### Example 6: Inhibition of Ca²⁺-Induced-ΔΨ Loss from Isolated Mitochondria by BH4 Domain of Bcl-x_{L}

The effects on ΔΨ loss were examined using the above-mentioned several kinds of the BH4 mutant polypeptides shown in Figure 13. Isolated mitochondria (1 mg/ml) were incubated with 20 µg/ml Bcl-x_{L} BH4 polypeptide in the presence of 40 µM Ca²⁺, and thereafter Rhodamine 123 intensity was continuously monitored. The results are shown in the left panel of Figure 14. In addition, isolated mitochondria (1 mg/ml) were incubated with 20 µg/ml BH4 polypeptide of Bcl-x_{L} and any of various mutant polypeptides in the presence of 40 µM Ca²⁺ for 15 minutes, and thereafter ΔΨ was measured by Rhodamine 123 intensity. The results are shown in the right panel of Figure 14.

As shown in Figures 14 and 15, the BH4 polypeptide of Bcl-x_{L} significantly inhibits Ca²⁺-induced ΔΨ loss and VDAC activity, although only at a molar concentration of about 25-fold higher than that of recombinant Bcl-x_{L}, consistent with the notion that other regions besides BH4 may increase the accessibility of VDAC. In fact, Bcl-2/Bcl-x_{L} BH1 mutant and BH2 mutant that influence the apoptotic activity indeed affect the ability of Bcl-2/Bcl-x_{L} to bind to VDAC. Further, all of the BH4 mutant polypeptides respectively showed their ability to inhibit VDAC activity (Figure 15) in proportion to their ability to inhibit mitochondrial ΔΨ (Figure 14).

It is suggested from these results that the BH4 domain of anti-apoptotic Bcl-x_{L} has an ability to inhibit apoptotic mitochondrial changes, which was in parallel to the ability to inhibit VDAC activity.

### Example 7: Inhibition of Apoptosis (Cell Death) by BH4 Domain

Since it was drawn up from the results of the above-mentioned Example 6 that even the BH4 polypeptide alone can have an ability to inhibit apoptotic mitochondrial changes, the possibility that the BH4 polypeptide might have an ability to inhibit apoptosis was further examined.

Therefore, in order to facilitate the transport of the BH4 polypeptide into cells, the above-mentioned TAT-BH4 polypeptide of 30 amino acid residues was synthesized, the polypeptide comprising N-terminal eosin-conjugated cystein and the PTD of HIV TAT protein fused to the Bcl-x_{L} BH4 polypeptide. HeLa cells were treated with 200 µM VP16 in the presence of the TAT-BH4 polypeptide, and then observed by an optical phase-contrast microscope and a fluorescence microscope, respectively. TAT-PTD has been known to facilitate the delivery of proteins into cells in a rapid, concentration-dependent manner. The results are shown in Figure 16, wherein the upper panel shows the cell morphology, and the lower panel shows the intracellular accumulation of the polypeptide.

Figure 16 shows that TAT-BH4 polypeptide which had been added to the culture medium was incorporated into the cells at a transfection efficiency of about 90%. Co-staining of the transfected cells with mitotracker revealed that the TAT-BH4 polypeptide was mainly localized at the mitochondria. As shown in Figures 16, 17 and 18, the TAT-BH4 polypeptide significantly inhibited VP16-induced apoptosis in a concentration-dependent manner, whereas TAT-only polypeptide did not significantly inhibit VP16-induced apoptosis at any concentrations. Also, BH4-only polypeptide showed no effect on VP16-induced apoptosis, as a result of an inefficient transport of the polypeptide into cells. It is shown from these findings that the TAT-BH4 polypeptide is sufficient to inhibit apoptotic cell death.

### Example 8- Evaluation of Anti-Apoptotic Activity of Various BH4 Fusion Polypeptides

TAT-BH4 polypeptides (BH4 fusion polypeptides) having the following amino acid sequences (SEQ ID NOs: 28 to 33) were synthesized in the same manner as in Example 2.

The resulting various BH4 fusion polypeptides were dissolved in H₂O to give aqueous solutions of 0.1 µg/ml or 1 µg/ml BH4 fusion polypeptide (apoptosis inhibitor). HeLa cells were pre-treated by bringing these aqueous solutions into contact with HeLa cells for 3 hours. HeLa cells were then treated with 200 µM VP16 to induce apoptosis. Each of the cells was stained with 1 µM Hoechst 33342, and an extent of apoptosis (viability) was calculated as a ratio of cells exhibiting nuclear fragmentation to total cells. The results are shown in Table 1.

**Table 1**

| BH4 Fusion | Viability (%) | |
|---|---|---|
| Polypeptide | 0.1 µg/ml | 1 µg/ml |
| 1 | 62.3 | 74.7 |
| 2 | 52.1 | 55.6 |
| 3 | 71.9 | 77.6 |
| 4 | 65.6 | 69.7 |
| 5 | 69.8 | 70.4 |
| 6 | 61.7 | 67.8 |
| Peptide | 46.5 | |
| No VP16, No peptide | 97.3 | |

It is seen from the results shown in Table 1 that the spacer group existing between the polypeptide (TAT) capable of exhibiting uptake action into cells and the BH4 domain of anti-apoptotic Bcl-2 family protein can be substituted with not only β-alanine but also with alanine or proline. In addition, it is seen that those having substitution of fourth glutamic acid with aspartic acid in the BH4 domain also possess anti-apoptotic activity at a practical level.

### SEQUENCE LIST FREE TEXT

SEQ ID NO: 1 shows consensus sequence of BH4 domain conserved in anti-apoptotic Bcl family protein.

In SEQ ID NO: 7, Xaa at position 1 shows eosin-conjugated cystein residue, and Xaa at position 11 shows β-Ala residue.

In SEQ ID NO: 8, Xaa at position 1 shows eosin-conjugated cystein residue, and Xaa at position 11 shows β-Ala residue.

SEQ ID NO: 11 is a sequence of a primer designed for preparing mutant bcl-x_{L} based on human bcl-x_{L} DNA sequence.

SEQ ID NO: 12 is a sequence of a primer designed for preparing mutant bcl-x_{L} based on human bcl-x_{L} DNA sequence.

SEQ ID NO: 13 is a sequence of a synthesized mutant bcl-x_{L} DNA sequence.

SEQ ID NO: 14 is an amino acid sequence for a synthesized mutant Bcl-x_{L} BH4 polypeptide.

SEQ ID NO: 15 is an amino acid sequence for a synthesized mutant Bcl-x_{L} BH4 polypeptide.

SEQ ID NO: 16 is an amino acid sequence for a synthesized mutant Bcl-x_{L} BH₄ polypeptide.

SEQ ID NO: 17 is an amino acid sequence for a synthesized mutant Bcl-x_{L} BH4 polypeptide.

SEQ ID NO: 18 is an amino acid sequence for a synthesized mutant Bcl-x_{L} BH4 polypeptide.

SEQ ID NO: 19 is an amino acid sequence for a synthesized mutant Bcl-x_{L} BH4 polypeptide.

SEQ ID NO: 20 is an amino acid sequence for a synthesized mutant Bcl-x_{L} BH4 polypeptide.

SEQ ID NO: 21 is an amino acid sequence for a synthesized mutant Bcl-x_{L} BH4 polypeptide.

SEQ ID NO: 23 is a polypeptide sequence resulting from introduction of eosin-conjugated cystein residue into N-terminal of a polypeptide TAT-PTD. In the sequence, wherein Xaa at position 1 shows eosin-conjugated cystein residue.

SEQ ID NO: 28 is an amino acid sequence of a synthesized BH4 fusion polypeptide. In the sequence, Xaa shows β-Ala residue.

SEQ ID NO: 29 is an amino acid sequence of a synthesized BH4 fusion polypeptide. In the sequence, Xaa shows β-Ala residue.

SEQ ID NO: 30 is an amino acid sequence of a synthesized BH4 fusion polypeptide.

SEQ ID NO: 31 is an amino acid sequence of a synthesized BH4 fusion polypeptide.

SEQ ID NO: 32 is an amino acid sequence of a synthesized BH4 fusion polypeptide.

SEQ ID NO: 33 is an amino acid sequence of a synthesized BH4 fusion polypeptide.

### INDUSTRIAL APPLICABILTTY

The BH4 fusion polypeptide of the present invention exhibits excellent effects such that each of mitochondrial ΔΨ loss and cytochrome *c* release can be inhibited, and that the uptake of the BH4 fusion polypeptide into cells can be efficiently carried out, so that apoptosis can be efficiently inhibited. In addition, since the apoptosis inhibitor of the present invention can inhibit apoptosis, it is useful as a therapeutic agent for AIDS, neurodegenerative disorders, osteomyelodysplasia, ischemic diseases, infectious multiple organ failure, fulminant hepatitis, diabetes and the like.

## Claims

1. A Bcl-2 homology 4 domain fusion polypeptide consisting of:
an amino acid sequence of a polypeptide or a derivative thereof which is capable of exhibiting uptake action into a cell; and
an amino acid sequence selected from the group consisting of:
(a) an amino acid sequence of SEQ ID NO: 1, 2, 3, 4, 9, 14, 15, 16, 17, 18, 19, 20, 21 or Ser 11 to Ser 30 of SEQ ID NO:29; and
(b) an amino acid sequence having substitution, deletion or insertion of one amino acid residue in the amino acid sequence of SEQ ID NO:1, 2, 3, 4, 9, 14, 15, 16, 17, 18, 19,20, 21 or Ser 11 to Ser 30 of SEQ ID NO:29,
wherein the Bcl-2 homology 4 domain fusion polypeptide is capable of inhibiting apoptosis.

2. The Bcl-2 homology 4 domain fusion polypeptide according to claim 1, wherein the derivative sequence is:
(i) an amino acid sequence having a chemically modified group of at least one residue in the amino acid sequence of the polypeptide capable of exhibiting uptake action into a cell, or
(ii) an amino acid sequence having substitution or insertion of at least one amino acid residue in the amino acid sequence of the polypeptide capable of exhibiting uptake action into a cell,
wherein the resulting polypeptide is capable of exhibiting uptake action into a cell.

3. The Bcl-2 homology 4 domain fusion polypeptide according to any one of claim 1 or 2, wherein the polypeptide capable of exhibiting uptake action into a cell is at least one kind selected from the group consisting of TAT from human immunodeficiency virus, p27 from HTLV-I, art/trs from HIV, VP22 from HSV-1 and Antp from *Drosophila.*

4. The Bcl-2 homology 4 domain fusion polypeptide according to any one of claims 1 to 3, wherein the amino acid sequence of the polypeptide capable of exhibiting uptake action into a cell is at least one kind selected from the group consisting of:
(i) a sequence comprising at least amino acid Nos: 49 to 57 of the amino acid sequence of SEQ ID NO:5;
(ii) a sequence comprising at least amino acid Nos:1 to 13 of the amino acid sequence of SEQ ID NO:24;
(iii) a sequence comprising at least amino acid Nos.:1 to 16 of the amino acid sequence of SEQ ID NO:25;
(iv) a sequence comprising at least amino acid Nos:1 to 301 of the amino acid sequence of SEQ ID NO:26; and
(v) a sequence comprising at least amino acid Nos:1 to 16 of the amino acid sequence of SEQ ID NO:27.

5. The Bcl-2 homology 4 domain fusion polypeptide according to any one of claims 1 to 4 consisting of
at least an amino acid sequence of SEQ ID NO:6; and
one kind selected from the group consisting of:
(a) an amino acid sequence of SEQ ID NO: 1, 2, 3, 4, 9, 14, 15, 16, 17, 18, 19, 20, 21 or Ser 11 to Ser 30 of SEQ ID NO:29; and
(b) an amino acid sequence having substitution, deletion or insertion of one amino acid residue in the amino acid sequence of SEQ ID NO:1, 2, 3, 4, 9, 14, 15, 16, 17, 18, 19, 20, 21 or Ser 11 to Ser 30 of SEQ ID NO:29,
wherein the Bcl-2 homology 4 domain fusion polypeptide is capable of inhibiting apoptosis.

6. The Bcl-2 homology 4 domain fusion polypeptide according to any one of claims 1 to 5 having at least one amino acid sequence selected from the group consisting of amino acid sequences of SEQ ID Nos.7, 8, 28, 29, 30, 31, 32 and 33.

7. The Bcl-2 homology 4 domain fusion polypeptide according to any one of claims 1 to 6, having a cystein residue at the amino terminal of the amino acid sequence of the polypeptide capable of exhibiting uptake action into a cell and β-alanine residue at carboxyl terminal.

8. A DNA encoding the Bcl-2 homology 4 domain fusion polypeptide according to any one of claims 1 to 7.

9. An apoptosis -inhibitor comprising the Bcl-2 homology 4 domain fusion polypeptide of any one of claims 1 to 7.

10. The apoptosis-inhibitor of claim 9 for use as a pharmaceutical composition.

11. Use of the apoptosis-inhibitor of claim 9 or 10 for the preparation of a pharmaceutical composition for treating an ischemic disease, AIDS, neurodegenerative disorders, osteomyelodysplasia, fulminant hepatitis, hepatic failures caused by addictions, infectious multiple organ failure or diabetes.

12. The use according to claim 11, wherein the ischemic disease is myocardial infarction.

13. A vector comprising the DNA of claim 8.

14. A host cell comprising the vector of claim 13.

15. A method of producing a polypeptide, comprising expressing a polypeptide encoded by the DNA of claim 8 and recovering said polypeptide.

## Patentansprüche

1. Bcl-2-Homologie-4-Domäne-Fusionspolypeptid bestehend aus:
einer Aminosäuresequenz eines Polypeptides oder eines Derivats davon, das Aufnahmeaktivität in eine Zelle zeigen kann; und
einer Aminosäuresequenz, ausgewählt aus der Gruppe bestehend aus:
(a) einer Aminosäuresequenz von SEQ ID NO: 1, 2, 3, 4, 9, 14, 15, 16, 17, 18, 19, 20, 21 oder Ser 11 bis Ser 30 von SEQ ID NO: 29; und
(b) einer Aminosäuresequenz mit Substitution, Deletion oder Insertion eines Aminosäurerests in der Aminosäuresequenz von SEQ ID NO: 1, 2, 3, 4, 9, 14, 15, 16, 17, 18, 19, 20, 21 oder Ser 11 bis Ser 30 von SEQ ID NO: 29,
wobei das Bcl-2-Homologie-Domäne-4-Fusionspolypeptid Apoptose hemmen kann.

2. Bcl-2-Homologie-4-Domäne-Fusionspolypeptid nach Anspruch 1, wobei die Derivatsequenz:
(i) eine Aminosäuresequenz mit einer chemisch modifizierten Gruppe von zumindest einem Rest in der Aminosäuresequenz des Polypeptids ist, das Aufnahmeaktivität in eine Zelle zeigen kann, oder
(ii) eine Aminosäuresequenz mit Substitution oder Insertion von mindestens einem Aminosäurerest in der Aminosäuresequenz des Polypeptids ist, das Aufnahmeaktivität in eine Zelle zeigen kann,
wobei das entstandene Polypeptid Aufnahmeaktivität in eine Zelle zeigen kann.

3. Bcl-2-Homologie-4-Domäne-Fusionspolypeptid nach einem der Ansprüche 1 oder 2, wobei das Polypeptid, das Aufnahmeaktivität in eine Zelle zeigen kann, mindestens eines ist, ausgewählt aus der Gruppe bestehend aus TAT vom menschlichen Immunschwächevirus, p27 von HTLV-I, art/trs von HIV, VP22 von HSV-1 und Antp von *Drosophila.*

4. Bcl-2-Homologie-4-Domäne-Fusionspolypeptid nach einem der Ansprüche 1 bis 3,
wobei die Aminosäuresequenz des Polypeptids, das Aufnahmeaktivität in eine Zelle zeigen kann, mindestens eines ist, das ausgewählt ist aus der Gruppe bestehend aus:
(i) einer Sequenz, umfassend mindestens die Aminosäurenummern 49 bis 57 der Aminosäuresequenz von SEQ ID NO: 5;
(ii) einer Sequenz, umfassend mindestens die Aminosäurenummern 1 bis 13 der Aminosäuresequenz von SEQ ID NO: 24;
(iii) einer Sequenz, umfassend mindestens die Aminosäurenummern 1 bis 16 der Aminosäuresequenz von SEQ ID NO: 25;
(iv) einer Sequenz, umfassend mindestens die Aminosäurenummern 1 bis 301 der Aminosäuresequenz von SEQ ID NO: 26; und
(v) einer Sequenz, umfassend mindestens die Aminosäurenummern 1 bis 16 der Aminosäuresequenz von SEQ ID NO. 27.

5. Bcl-2-Homologie-4-Domäne-Fusionspolypeptid nach einem der Ansprüche 1 bis 4 bestehend aus
mindestens einer Aminosäuresequenz von SEQ ID NO: 6; und
einer, ausgewählt aus der Gruppe bestehend aus:
(a) einer Aminosäuresequenz von SEQ ID NO: 1, 2, 3, 4, 9, 14, 15, 16, 17, 18, 19, 20, 21 oder Ser 11 bis Ser 30 von SEQ ID NO: 29; und
(b) einer Aminosäuresequenz mit Substitution, Deletion oder Insertion eines Aminosäurerests in der Aminosäuresequenz von SEQ ID NO: 1, 2, 3, 4, 9, 14, 15, 16, 17, 18, 19, 20, 21 oder Ser 11 bis Ser 30 von SEQ ID NO: 29,
wobei das Bcl-2-Homologie-4-Domäne-Fusionspolypeptid Apoptose hemmen kann.

6. Bcl-2-Homologie-4-Domäne-Fusionspolypeptid nach einem der Ansprüche 1 bis 5 mit mindestens einer Aminosäuresequenz ausgewählt aus der Gruppe bestehend aus den Aminosäuresequenzen von SEQ ID NO: 7, 8, 28, 29, 30, 31, 32 und 33.

7. Bcl-2-Homologie-4-Domäne-Fusionspolypeptid nach einem der Ansprüche 1 bis 6 mit einem Cysteinrest am Amino-Terminus der Aminosäuresequenz des Polypeptids, das Aufnahmeaktivität in eine Zellen zeigen kann, und einem β-Alaninrest am CarboxyTerminus.

8. DNA, die das Bcl-2-Homologie-4-Domäne-Fusionspolypeptid nach einem der Ansprüche 1 bis 7 codiert.

9. Apoptoseinhibitor, umfassend das Bcl-2-Homologie-4-Domäne-Fusionspolypeptid nach einem der Ansprüche 1 bis 7.

10. Apoptoseinhibitor nach Anspruch 9 zur Verwendung als Arzneimittel.

11. Verwendung des Apoptoseinhibitors nach Anspruch 9 oder 10 für die Herstellung eines Arzneimittels zur Behandlung einer ischämischen Erkrankung, von AIDS, neurodegenerativen Störungen, Osteomyelodysplasie, fulminanter Hepatitis, Leberversagen, verursacht durch Suchten, infektiösem multiplen Organversagen oder Diabetes.

12. Verwendung nach Anspruch 11, wobei die ischämische Erkrankung ein Herzinfarkt ist.

13. Vektor, umfassend die DNA nach Anspruch 8.

14. Wirtszelle, umfassend den Vektor nach Anspruch 13.

15. Verfahren zur Herstellung eines Polypeptids, umfassend das Exprimieren des Polypeptids, das von der DNA nach Anspruch 8 codiert wird, und das Gewinnen des Polypeptids.

## Revendications

1. Polypeptide de fusion avec un domaine homologue 4 de Bcl-2 constitué de :
une séquence d'acides aminés d'un polypeptide ou d'un dérivé de celui-ci qui
est capable de présenter une action de recapture dans une cellule ; et
une séquence d'acides aminés choisie parmi le groupe constitué de :
(a) une séquence d'acides aminés de SEQ ID NO :1, 2, 3, 4, 9, 14, 15, 16, 17, 18, 19, 20, 21, ou de Ser 11 à Ser 30 de SEQ ID NO :29 ; et
(b) une séquence d'acides aminés ayant une substitution, une délétion ou une insertion d'un résidu acide aminé dans la séquence d'acides aminé de SEQ ID NO : 1, 2, 3, 4, 9, 14, 15, 16, 17, 18, 19, 20, 21 ou de Ser 11 à Ser 30 de SEQ ID NO : 29,
le polypeptide de fusion avec un domaine homologue 4 de Bcl-2 étant capable d'inhiber l'apoptose.

2. Polypeptide de fusion avec un domaine homologue 4 de Bcl-2 selon la revendication 1, dans lequel la séquence du dérivé est :
(i) une séquence d'acides aminés ayant au moins un résidu avec un groupement modifié chimiquement dans la séquence d'acides aminés du polypeptide capable de présenter une action de recapture dans une cellule, ou
(ii) une séquence d'acides aminés ayant une substitution ou une insertion d'au moins un résidu acide aminé dans la séquence d'acides aminés du polypeptide capable de présenter une action de recapture dans une cellule,
le polypeptide résultant étant capable de présenter une action de recapture dans une cellule.

3. Polypeptide de fusion avec un domaine homologue 4 de Bcl-2 selon l'une quelconque des revendications 1 ou 2, dans lequel le polypeptide capable de présenter une action de recapture dans une cellule est au moins d'un type choisi parmi le groupe constitué de TAT du virus humain de l'immunodéficience, de p27 de HTLV-1, de art/trs du VIH, de VP22 de HSV-1 et de Antp de *Drosophila.*

4. Polypeptide de fusion avec un domaine homologue 4 de Bcl-2 selon l'une quelconque des revendications 1 à 3, dans lequel la séquence d'acides aminés du polypeptide capable de présenter une action de recapture dans une cellule est au moins d'un type choisi parmi le groupe constitué de :
(i) une séquence comprenant au moins les acides aminés Nos : 49 à 57 de la séquence d'acides aminés SED ID NO :5 ;
(ii) une séquence comprenant au moins les acides aminés Nos : 1 à 13 de la séquence d'acides aminés SED ID NO :24 ;
(iii) une séquence comprenant au moins les acides aminés Nos : 1 à 16 de la séquence d'acides aminés SED ID NO :25 ;
(iv) une séquence comprenant au moins les acides aminés Nos : 1 à 301 de la séquence d'acides aminés SED ID NO :26 ; et
(v) une séquence comprenant au moins les acides aminés Nos : 1 à 16 de la séquence d'acides aminés SED ID NO :27.

5. Polypeptide de fusion avec un domaine homologue 4 de Bcl-2 selon l'une quelconque des revendications 1 à 4 constitué de au moins une séquence d'acides aminés de SEQ ID NO :6 ; et un type choisi parmi le groupe constitué de :
(a) une séquence d'acides aminés de SEQ ID NO : 1, 2, 3, 4, 9, 14, 15, 16, 17, 18, 19, 20, 21, ou de Ser 11 à Ser 30 de SEQ ID NO:29; et
(b) une séquence d'acides aminés ayant une subtitution, délétion ou insertion d'un résidu acide aminé dans la séquence d'acides aminés de SEQ ID NO : 1, 2, 3, 4, 9, 14, 15, 16, 17, 18, 19, 20, 21 ou de Ser 11 à Ser 30 de SEQ ID NO : 29,
dans lequel le polypeptide de fusion avec un domaine homologue 4 de Bcl-2 est capable d'inhiber l'apoptose.

6. Polypeptide de fusion avec un domaine homologue 4 de Bcl-2 selon l'une quelconque des revendications 1 à 5 ayant au moins une séquence d'acides aminés choisie parmi le groupe constitué des séquences d'acides aminés de SEQ ID Nos. 7, 8, 28, 29, 30, 31, 32 et 33.

7. Polypeptide de fusion avec un domaine homologue 4 de Bcl-2 selon l'une quelconque des revendications 1 à 6, ayant un résidu cystéine à l'extrémité amino terminale de la séquence d'acides aminés du polypeptide capable de présenter une action de recapture dans une cellule et un résidu β-alanine à l'extrémité carboxy terminale.

8. ADN codant le polypeptide de fusion avec un domaine homologue 4 de Bcl-2 selon l'une quelconque des revendications 1 à 7.

9. Inhibiteur de l'apoptose comprenant le polypeptide de fusion avec un domaine homologue 4 de Bcl-2 selon l'une quelconque des revendications 1 à 7.

10. Inhibiteur de l'apoptose selon la revendication 9 pour une utilisation en tant que composition pharmaceutique.

11. Utilisation de l'inhibiteur de l'apoptose selon la revendication 9 ou 10 pour la préparation d'une composition pharmaceutique pour traiter une maladie ischémique, le SIDA, des désordres neurodégénératifs, une ostéomyélodisplasie, un ictère grave, des insuffisances hépatiques causées par addictions, des insuffisances d'organes multiples d'origine infectieuse ou des diabètes.

12. Utilisation selon la revendication 11, dans laquelle la maladie ischémique est un infarctus du myocarde.

13. Vecteur comprenant l'ADN selon la revendication 8.

14. Cellule hôte comprenant le vecteur selon la revendication 13.

15. Méthode de production d'un polypeptide, comprenant l'expression d'un polypeptide codé par l'ADN selon la revendication 8 et la récupération dudit polypeptide.


### SEQUENCE LISTING

<110> SHIONOGI & CO., LTD.
<120> A BH4 fusion polypeptide
<130> 00-061-PCT
<150> JP 11-371449
   <151> 1999-12-27
<160> 33
<170> PatentIn Ver. 2.0
<210> 1
   <211> 20
   <212> PRT
   <213> Artificial Sequence
<220>
   <223> Consensus sequence of BH4 domain conserved in anti-apoptotic Bcl-2 family proteins.
<220>
   <223> Xaa at position 7, 10, and 11 can be any kinds of amino acid resid ues.
<400> 1
   Asp Asn Arg Glu Leu Val Xaa Asp Phe Xaa Xaa Tyr Lys Leu Ser Gln Lys
   1 5 10 15
   Gly Tyr Ser
   20
<210> 2
   <211> 24
   <212> PRT
   <213> Homo sapiens
<400> 2
   Thr Gly Tyr Asp Asn Arg Glu Ile Val Met Lys Tyr Ile His Tyr Lys Leu
   1 5 10 15
   Ser Gln Arg Gly Tyr Glu Trp
   20
<210> 3
   <211> 20
   <212> PRT
   <213> Homo sapiens
<400> 3
   Ser Asn Arg Glu Leu Val Val Asp Phe Leu Ser Tyr Lys Leu Ser Gln Lys
   1 5 10 15
   Gly Tyr Ser
   20
<210> 4
   <211> 20
   <212> PRT
   <213> Homo sapiens
<400> 4
   Asp Thr Arg Ala Leu Val Ala Asp Phe Val Gly Tyr Lys Leu Arg Gln
   1 5 10 15
   Lys Gly Tyr Val
   20
<210> 5
   <211> 86
   <212> PRT
   <213> Homo sapiens
<400> 5
   Met Glu Pro Val Asp Pro Arg Leu Glu Pro Trp Lys His Pro Gly Ser Gln
   1 5 10 15
   Pro Lys Thr Ala Cys Thr Asn Cys Tyr Cys Lys Lys Cys Cys Phe His Cys
   20 25 30
   Gln Val Cys Phe Ile Thr Lys Ala Leu Gly Ile Ser Tyr Gly Arg Lys Lys
   35 40 45 50
   Arg Arg Gln Arg Arg Arg Ala His Gln Asn Ser Gln Thr His Gln Ala Ser
   55 60 65
   Leu Ser Lys Gln Pro Thr Ser Gln Pro Arg Gly Asp Pro Thr Gly Pro Lys
   70 75 80 85
   Glu
<210> 6
   <211> 8
   <212> PRT
   <213> Homo sapiens
<400> 6
<210> 7
   <211> 35
   <212> PRT
   <213> Homo sapiens
<220>
   <223> Xaa at position 1 is eosin-conjugated cystein residue.
<220>
   <223> Xaa at position 11 is bAla residue.
<400> 7
   Xaa Arg Lys Lys Arg Arg Gln Arg Arg Arg Xaa Thr Gly Tyr Asp Asn Arg
   1 5 10 15
   Glu Ile Val Met Lys Tyr Ile His Tyr Lys Leu Ser Gln Arg Gly Tyr Glu
   20 25 30
   Trp
   35
<210> 8
   <211> 31
   <212> PRT
   <213> Homo sapiens
<220>
   <223> Xaa at position 1 is eosin-conjugated cystein residue.
<220>
   <223> Xaa at position 11 is bAla residue.
<440> 8
<210> 9
   <211> 20
   <212> PRT
   <213> Caenorhabditis elegans
<400> 9
   Asp Ile Glu Gly Phe Val Val Asp Tyr Phe Thr His Arg Ile Arg Gln
   1 5 10 15
   Asn Gly Met Glu
   20
<210> 10
   <211> 233
   <212> PRT
   <213> Homo sapiens
<400> 10
<210> 11
   <211> 47
   <212> DNA
   <213> Artificial Sequence
<220>
   <223> Designed nucleotide for synthesizing a mutant bcl-x _{L} based on hum an bcl-x _{L} DNA sequence.
<400> 11
   cggaattcat gtcttggagt cagtttagtg atgtggaaga gaacagg 47
<210> 12
   <211> 38
   <212> DNA
   <213> Artificial Sequence
<220>
   <223> Designed nucleotide for synthesizing mutant bcl-x _{L} based on human bcl-x _{L} DNA sequence.
<400> 12
   cggaattctg gtcatttccg actgaagagt gagcccag 38
<210> 13
   <211> 636
   <212> DNA
   <213> Artificial Sequence
<220>
   <223> Synthesized DNA for mutant bcl-x_{L}
<221> CDS
   <222> (1)..(636)
<400> 13
   atg tct tgg agt cag ttt agt gat gtg gaa gag aac agg act gag gcc 48
   Met Ser Trp Ser Gln Phe Ser Asp Val Glu Glu Asn Arg Thr Glu Ala
   1 5 10 15
   cca gaa ggg act gaa tcg gag atg gag acc ccc agt gcc atc aat ggc 96
   Pro Glu Gly Thr Glu Ser Glu Met Glu Thr Pro Ser Ala Ile Asn Gly
   20 25 30
   aac cca tcc tgg cac ctg gca gac agc ccc gcg gtg aat gga gcc act 144
   Asn Pro Ser Trp His Leu Ala Asp Ser Pro Ala Val Asn Gly Ala Thr
   35 40 45
   gcg cac agc agc agt ttg gat gcc cgg gag gtg atc ccc atg gca gca 192
   Ala His Ser Ser Ser Leu Asp Ala Arg Glu Val Ile Pro Met Ala Ala
   50 55 60
   gta aag caa gcg ctg agg gag gca ggc gac gag ttt gaa ctg cgg tac 240
   Val Lys Gln Ala Leu Arg Glu Ala Gly Asp Glu Phe Glu Leu Arg Tyr
   65 70 75 80
   cgg cgg gca ttc agt gac ctg aca tcc cag ctc cac atc acc cca ggg 288
   Arg Arg Ala Phe Ser Asp Leu Thr Ser Gln Leu His Ile Thr Pro Gly
   85 90 95
   aca gca tat cag agc ttt gaa cag gta gtg aat gaa ctc ttc cgg gat 336
   Thr Ala Tyr Gln Ser Phe Glu Gln Val Val Asn Glu Leu Phe Arg Asp
   100 105 110
   ggg gta aac tgg ggt cgc att gtg gcc ttt ttc tcc ttc ggc ggg gca 384
   Gly Val Asn Trp Gly Arg Ile Val Ala Phe Phe Ser Phe Gly Gly Ala
   115 120 125
   ctg tgc gtg gaa agc gta gac aag gag atg cag gta ttg gtg agt cgg 432
   Leu Cys Val Glu Ser Val Asp Lys Glu Met Gln Val Leu Val Ser Arg
   130 135 140
   atc gca gct tgg atg gcc act tac ctg aat gac cac cta gag cct tgg 480
   Ile Ala Ala Trp Met Ala Thr Tyr Leu Asn Asp His Leu Glu Pro Trp
   145 150 155 160
   atc cag gag aac ggc ggc tgg gat act ttt gtg gaa ctc tat ggg aac 528
   Ile Gln Glu Asn Gly Gly Trp Asp Thr Phe Val Glu Leu Tyr Gly Asn
   165 170 175
   aat gca gca gcc gag agc cga aag ggc cag gaa cgc ttc aac cgc tgg 576
   Asn Ala Ala Ala Glu Ser Arg Lys Gly Gln Glu Arg Phe Asn Arg Trp
   180 185 190
   ttc ctg acg ggc atg act gtg gcc ggc gtg gtt ctg ctg ggc tca ctc 624
   Phe Leu Thr Gly Met Thr Val Ala Gly Val Val Leu Leu Gly Ser Leu
   195 200 205
   ttc agt cgg aaa 636
   Phe Ser Arg Lys
   210
<210> 14
   <211> 20
   <212> PRT
   <213> Artificial Sequence
<220>
   <223> Synthesized peptide for mutant Bcl-x _{L} BH4.
<400> 14
   Ser Asn Arg Glu Val Phe Val Asp Phe Leu Ser Tyr Lys Leu Ser Gln Lys
   1 5 10 15
   Gly Tyr Ser
   20
<210> 15
   <211> 20
   <212> PRT
   <213> Artificial Sequence
<220>
   <223> Synthesized peptide for mutant Bcl-x _{L} BH4.
<400> 15
   Ser Asn Arg Glu Gly Gly Val Asp Phe Leu Ser Tyr Lys Leu Ser Gln Lys
   1 5 10 15
   Gly Tyr Ser
   20
<210> 16
   <211> 18
   <212> PRT
   <213> Artificial Sequence
<220>
   <223> Synthesized peptide for mutant Bcl-x _{L} BH₄.
<400> 16
   Ser Asn Arg Glu Val Asp Phe Leu Ser Tyr Lys Leu Ser Gln Lys Gly Tyr
   1 5 10 15
   Ser
<210> 17
   <211> 20
   <212> PRT
   <213> Artificial Sequence
<220>
   <223> Synthesized peptide for mutant Bcl-x _{L} BH4.
<400> 17
   Ser Asn Arg Glu Leu Val Gly Gly Phe Leu Ser Tyr Lys Leu Ser Gln Lys
   1 5 10 15
   Gly Tyr Ser
   20
<210> 18
   <211> 20
   <212> PRT
   <213> Artificial Sequence
<220>
   <223> Synthesized peptide for mutant Bcl-x _{L} BH4.
<400> 18
   Ser Asn Arg Glu Leu Val Val Asp Gly Gly Ser Tyr Lys Leu Ser Gln Lys
   1 5 10 15
   Gly Tyr Ser
   20
<210> 19
   <211> 18
   <212> PRT
   <213> Artificial Sequence
<220>
   <223> Synthesized peptide for mutant Bcl-x _{L} BH4.
<400> 19
   Ser Asn Arg Glu Leu Val Val Asp Ser Tyr Lys Leu Ser Gln Lys Gly Tyr
   1 5 10 15
   Ser
<210> 20
   <211> 20
   <212> PRT
   <213> Artificial Sequence
<220>
   <223> Synthesized peptide for mutant Bcl-x _{L} BH4.
<400> 20
   Ser Asn Arg Glu Leu Val Val Gly Phe Leu Ser Tyr Lys Leu Ser Gln Lys
   1 5 10 15
   Gly Tyr Ser
   20
<210> 21
   <211> 20
   <212> PRT
   <213> Artificial Sequence
<220>
   <223> Synthesized peptide for mutant Bcl-x _{L} BH4.
<400> 21
   Ser Asn Arg Glu Leu Val Val Asp Phe Leu Ser Tyr Lys Trp Ser Gln Lys
   1 5 10 15
   Gly Tyr Ser
   20
<210> 22
   <211> 24
   <212> PRT
   <213> Homo sapiens
<400> 22
   Val Ala Gln Asp Thr Glu Glu Val Phe Arg Ser Tyr Val Phe Tyr Arg His
   1 5 10 15
   Gln Gln Glu Gln Glu Ala Glu
   20
<210> 23
   <211> 10
   <212> PRT
   <213> Artficial Sequence
<220>
   <223> Synthesized peptide for BH4 fusion peptide
<220>
   <223> Xaa at position 1 is eosin-conjugated cystein residue.
<400> 23
   Xaa Arg Lys Lys Arg Arg Gln Arg Arg Arg
   1               5                   10
<210> 24
   <211> 13
   <212> PRT
   <213> Human T lymphotropic virus
<400> 24
   Lys Thr Arg Arg Arg Pro Arg Arg Ser Gln Arg Lys Arg
   1               5                   10
<210> 25
   <211> 16
   <212> PRT
   <213> Human T lymphotropic virus
<400> 25
   Arg Gln Ala Arg Arg Asn Arg Arg Arg Arg Trp Arg Glu Arg Gln Arg
   1               5                   10                  15
<210> 26
   <211> 301
   <212> PRT
   <213> herpes simplex virus type 1
<400> 26
   Met Thr Ser Arg Arg Ser Val Lys Ser Gly Pro Arg Glu Val Pro Arg
   1               5                   10                  15
   Asp Glu Tyr Glu Asp Leu Tyr Tyr Thr Pro Ser Ser Gly Met Ala Ser
   20 25 30
   Pro Asp Ser Pro Pro Asp Thr Ser Arg Arg Gly Ala Leu Gln Thr Arg
   35 40 45
   Ser Arg Gln Arg Gly Glu Val Arg Phe Val Gln Tyr Asp Glu Ser Asp
   50 55 60
   Tyr Ala Leu Tyr Gly Gly Ser Ser Ser Glu Asp Asp Glu His Pro Glu
   65 70 75 80
   Val Pro Arg Thr Arg Arg Pro Val Ser Gly Ala Val Leu Ser Gly Pro
   85 90 95
   Gly Pro Ala Arg Ala Pro Pro Pro Pro Ala Gly Ser Gly Gly Ala Gly
   100 105 110
   Arg Thr Pro Thr Thr Ala Pro Arg Ala Pro Arg Thr Gln Arg Val Ala
   115 120 125
   Thr Lys Ala Pro Ala Ala Pro Ala Ala Glu Thr Thr Arg Gly Arg Lys
   130 135 140
   Ser Ala Gln Pro Glu Ser Ala Ala Leu Pro Asp Ala Pro Ala Ser Thr
   145 150 155 160
   Ala Pro Thr Arg Ser Lys Thr Pro Ala Gln Gly Leu Ala Arg Lys Leu
   165 170 175
   His Phe Ser Thr Ala Pro Pro Asn Pro Asp Ala Pro Trp Thr Pro Arg
   180 185 190
   Val Ala Gly Phe Asn Lys Arg Val Phe Cys Ala Ala Val Gly Arg Leu
   195 200 205
   Ala Ala Met His Ala Arg Met Ala Ala Val Gln Leu Trp Asp Met Ser
   210 215 220
   Arg Pro Arg Thr Asp Glu Asp Leu Asn Glu Leu Leu Gly Ile Thr Thr
   225 230 235 240
   Ile Arg Val Thr Val Cys Glu Gly Lys Asn Leu Leu Gln Arg Ala Asn
   245 250 255
   Glu Leu Val Asn Pro Asp Val Val Gln Asp Val Asp Ala Ala Thr Ala
   260 265 270
   Thr Arg Gly Arg Ser Ala Ala Ser Arg Pro Thr Glu Arg Pro Arg Ala
   275 280 285
   Pro Ala Arg Ser Ala Ser Arg Pro Arg Arg Pro Val Glu
   290 295 300
<210> 27
   <211> 16
   <212> PRT
   <213> Drosophila
<400>27
   Arg Gln Ile Lys Ile Phe Phe Gln Asn Arg Arg Met Lys Phe Lys Lys
   1 5 10 15
<210> 28
   <211> 30
   <212> PRT
   <213> Artificial Sequence
<220>
   <223> Synthesized peptide for BH4 fusion peptide
<220>
   <223> Xaa at position 10 is bAla residue.
<400> 28
   Arg Lys Lys Arg Arg Gln Arg Arg Arg Xaa Ser Asn Arg Glu Leu Val
   1 5 10 15
   Val Asp Phe Leu Ser Tyr Lys Leu Ser Gln Lys Gly Tyr Ser
   20 25 30
<210> 29
   <211> 30
   <212> PRT
   <213> Artificial Sequence
<220>
   <223> Synthesized peptide for BH4 fusion peptide
<220>
   <223> Xaa at position 10 is bAla residue.
<400>29
   Arg Lys Lys Arg Arg Gln Arg Arg Arg Xaa Ser Asn Arg Asp Leu Val
   1 5 10 15
   Val Asp Phe Leu Ser Tyr Lys Leu Ser Gln Lys Gly Tyr Ser
   20 25 30
<210> 30
   <211 > 30
   <212> PRT
   <213> Artificial Sequence
<220>
   <223> Synthesized peptide for BH4 fusion peptide
<400> 30
   Arg Lys Lys Arg Arg Gln Arg Arg Arg Ala Ser Asn Arg Glu Leu Val
   1 5 10 15
   Val Asp Phe Leu Ser Tyr Lys Leu Ser Gln Lys Gly Tyr Ser
   20 25 30
<210> 31
   <211> 30
   <212> PRT
   <213> Artificial Sequence
<220>
   <223> Synthesized peptide for BH4 fusion peptide
<400> 31
   Arg Lys Lys Arg Arg Gln Arg Arg Arg Ala Ser Asn Arg Asp Leu Val
   1 5 10 15
   Val Asp Phe Leu Ser Tyr Lys Leu Ser Gln Lys Gly Tyr Ser
   20 25 30
<210> 32
   <211> 30
   <212> PRT
   <213> Artificial Sequence
<220>
   <223> Synthesized peptide for BH4 fusion peptide
<400> 32
   Arg Lys Lys Arg Arg Gln Arg Arg Arg Pro Ser Asn Arg Glu Leu Val
   1 5 10 15
   Val Asp Phe Leu Ser Tyr Lys Leu Ser Gln Lys Gly Tyr Ser
   20 25 30
<210> 33
   <211> 30
   <212> PRT
   <213> Artificial Sequence
<220>
   <223> Synthesized peptide for BH4 fusion peptide
<400> 33
   Arg Lys Lys Arg Arg Gln Arg Arg Arg Pro Ser Asn Arg Asp Leu Val
   1 5 10 15
   Val Asp Phe Leu Ser Tyr Lys Leu Ser Gln Lys Gly Tyr Ser
   20 25 30